# EUROPEAN PATENT APPLICATION

(11) **EP 4 389 754 A1**
(43) Date of publication of application: **26.06.2024**
(21) Application number: 22215963.4
(22) Date of filing: 22.12.2022
(51) Int. Cl.: C07F 5/02, C09K 11/06, H10K 85/00, C07D 498/08, C07F 5/00

(54) **METAL-ORGANIC COORDINATION COMPOUND AND METHOD FOR PRODUCING THE SAME**

(71) Applicant: beeOLED GmbH, 01257 Dresden (DE)
(72) Inventor: SENKOVSKYY, Volodymyr, 01187 Dresden (DE)
(74) Representative: Altmann, Andreas

(57) **Abstract**

In metal-organic coordination compound, the coordination compound comprises one lanthanide ion coordinated by a polycyclic organic ligand having the formula 1-1 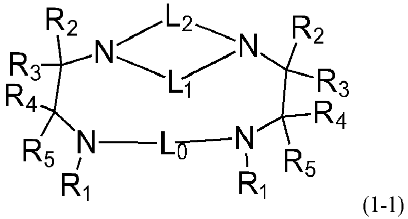 with
• **L₀, L₁, L₂** being independently each a divalent organic group formed by removing two hydrogen atoms from a substituted or non-substituted alkane, arylalkane, heteroarylalkane, arene, heteroarene, alkylarene, alkylheteroarene, dialkylarene or dialkylheteroarene, wherein alkane or alkyl can be interrupted by one or more oxygen or boron atoms, and with
• the sum of the number of atoms in the shortest sequences of atoms in **L₁**, and **L₂** between the two linkages of -L₁- and -L₂- and the remainder of the polycyclic ligand being less than 16 atoms, and
• **R₂** to **R₅** independently in each occurrence representing hydrogen, deuterium, halogen, or an organyl group, and
• **R₁** representing hydrogen, deuterium, or an organyl group.

## Description

### Technical Field

The invention concerns a metal-organic coordination compound, wherein the coordination compound comprises at least one lanthanide coordinated by a polycyclic organic ligand, a mixture containing the metal-organic coordination compound, a polymeric compound, wherein the coordination compound is covalently attached to the polymer backbone, an organic electronic device containing them, and methods for forming the same.

### Background

Many applications relevant physical and in particular opto electronic processes make use of lanthanide ions with their chemically well shielded inner f-f transitions. One of the most important applications are electroluminescent devices based on organic light emitting diodes (OLEDs) for example for consumer displays.

Indeed, those intrametallic lanthanide transitions possess significant advantages over conventionally used organic fluorescent emitters in terms of efficiency. For the latter, quantum statistical selection rules dictate that no more than 25% of the input power can be converted into useful light, 75% of the invested electrical power is wasted, which renders those fluorescent emitter materials intrinsically very inefficient. To overcome this, incorporation of quantum mechanical heavy metal effect into the emitter molecules, by introducing d-metal elements such as Iridium, Osmium, Gold or Platinum, has been proposed. The presence of heavy metal elements softens the selection rules for the excited states resulting in high internal light generation efficiencies; known as phosphorescence (Ph). Similarly, thermally activated delayed fluorescence (TADF) has been used wherein thermal energy from the environment is harvested to convert non-emissive states back into emissive ones, resulting in high light generation efficiencies.

However, both TADF and phosphorescent emitters suffer from severe chemical instabilities. Here, during excitation, especially with high energetic blue light, the chemical bonds are weakened, giving rise to chemical cleavage and in consequence short operational lifetime in OLED device. Neither TADF nor phosphorescent blue emitting materials can be used for display applications, as otherwise the blue spectral component would fade away after prolonged operation times which is known as burn-in.

On the other hand, many lanthanide transitions are intrinsically 100% efficient giving rise to maximum achievable light generation efficiency. At the same time, those transitions are of intra metallic nature. Thus, it is fundamentally impossible to cleave any organic bonds during light generation. Therefore, the use of lanthanide emitters promises the combination of high efficiency and high chemical stability, which is absent in current OLED emitter designs.

Indeed, emitters based on various lanthanides have been extensively used in OLEDs. For example, OLEDs based on intra-atomic transitions in the sky-blue, green, and red spectral region based on Thulium (Tm3+), Terbium (Ter3+) and Europium (Eu3+) respectively have been demonstrated. The luminescence spectra of those lanthanide emitters are characterized by a plurality of individually narrow emission lines spaced over a very wide spectral region. However, for display applications, the emission spectra of the primary red, green, and blue colors have to be narrow in order to allow for the highest color purities. Otherwise, color filters are used to sharpen the emission spectra, compromising efficiency. Adding to this, the excited state lifetimes of those above f-f transition type emitters, typically around one millisecond, is too long to allow for fast display refresh rates. Besides such long excited state lifetimes lead to bimolecular quenching of the excited states at high driving currents, again compromising efficiency.

The above limitations are a direct consequence of the nature of the intrametallic f-f transitions. In fact, those limitations are lifted for d-f lanthanide transition, i.e. parity allowed transitions between different orbitals, in particular for Europium (Eu2+), Cerium (Ce3+) and Ytterbium (Yb2+), which possess desired short excited state lifetimes of the order of one microsecond or less.

Additionally, the d-orbital involved in the optical transition is not fully shielded from the outside, and by careful engineering of the environment deep blue, narrow emission spectra are readily possible, especially for Yb(II) and Eu(II). Less color pure doublets are still obtainable in case of Ce(III).

With respect to electroluminescent emitters, the obvious way to apply the lanthanide cations is as part of a metal organic complex. Here the organic ligand acts as chemical environment for the lanthanide cation and its electronic structure thus influences the emission color of the complex via interaction with the d orbital of the metals. While tuning the color into the desired spectral region, the ligand should refrain from significant excited state relaxation as this is known to lower the light emission efficiency. Thus, the ideal ligand is structurally rigid as opposed to being flexible.

The preferred oxidation state of all lanthanide metals is trivalent. Thus, a major obstacle particularly for the application of divalent lanthanides, such as the color pure Eu(II), is their chemical instability, and -in particular- their tendency to oxidize under normal ambient conditions. Therefore, the application of divalent lanthanide requires sufficient stabilization of the cation.

According to WO 2011/090977, oxidation is prevented by incorporating soft coordinating ligands, such as sulfur or aromatic aryl or heteroaryl rings into the coordination sphere of the central metal. Thereby oxidation stability of the central metal may be observed. However, providing such a soft, asymmetrical coordination environment leads to polarization effects in the excited state of the central metal. In other words, the originally pure intra-metallic transition on the central metal ion becomes partly of metal-to-ligand charge transfer (MLCT) type character. Consequently, the originally deep blue and spectrally pure emission shifts substantially to the green and red spectral region and broadens, which renders the application of those metal coordination compounds in opto-electronic devices undesirable.

In Chem. Commun., 2018, 54, pages 4545-4548, nitrogen-containing (macro)circular molecules with Eu(II) and their application in OLED device are disclosed. Again the central cation is heavily electronically stabilized by a plurality of soft nitrogen atoms and as such only emitts in the undesirable yellow-green spectral region.

In WO2021244801A1, stable Eu(II) and Yb(II) emitters using macrocyclic ligands are disclosed. Here, the stabilization is achieved by geometrical stabilization, in particular by careful shielding of the central cations from the outside using inert side groups. However, whilst those emitters do preserve the desired deep blue emission color, it remains chemically difficult to selectively add further side groups that are needed for high performance in OLED applications. In this context molecules without non-covalently attached anions are preferred as otherwise anion drift during OLED operation will compromise the device lifetime. Indeed, such anion attachment could be achieved using a complicated synthesis route resulting in mixtures of isomers. However, as a consequence of the nature of the shielding groups, the anions are located relatively far away from the central cation, leading to high dipole moments, which in turn may make it difficult to process such materials using thermal sublimation.

Thus, no coordination compounds are known that do keep the desired properties of the divalent lanthanides, in particular its deep blue color, preferably having no non-covalently attached anions, and are preferably sufficiently stable to be processed by thermal sublimation.

An object underlying the present invention is to provide an electroluminescent coordination compound which shows a deep blue, saturated emission profile combined with high operational stability in an OLED device.

The object is achieved according to the present invention by a metal-organic coordination compound, wherein the coordination compound comprises one lanthanide ion coordinated by a polycyclic organic ligand having the formula 1-1 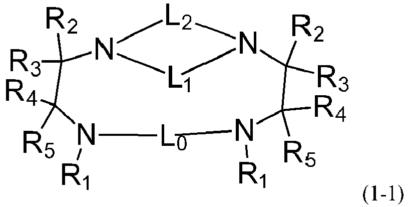 with
- **L₀, L₁, L₂** being independently each a divalent organic group formed by removing two hydrogen atoms from a substituted or non-substituted alkane, arylalkane, heteroarylalkane, arene, heteroarene, alkylarene, alkylheteroarene, dialkylarene or dialkylheteroarene, wherein alkane or alkyl can be interrupted by one or more oxygen or boron atoms, and with
- the sum of the number of atoms in the shortest sequences of atoms in **L₁**, and **L₂** between the two linkages of -L₁- and -L₂- and the remainder of the polycyclic ligand being less than 16 atoms, and
- **R₂** to **R₅** independently in each occurrence representing hydrogen, deuterium, halogen, or an organyl group, and
**R₁** representing hydrogen, deuterium, or an organyl group.

In particular, it was found that in prior art coordination compounds the cavity size of the mostly symmetrical polycyclic organic ligand did in fact not perfectly match the central cation, as it was often far too large. Too large diameters of the cavity have been mostly employed, as the polycyclic organic ligands where mostly based on ring diameters containing 18 atoms. It is thus part of this invention to provide geometrical stabilization by means of a rigid polycyclic organic ligand with a cavity size perfectly matching the size of the central cation. Additionally, the ligand contains some donor atoms to achieve electronic stabilization and deep blue color point. Additionally, it is a feature of the present invention to provide molecules where neither the molecule itself nor parts of it drift within the OLED device upon application of an electrical field, which is known to seriously compromise the stability of such an OLED device and would often prevent application of such OLED devices in consumer electronics. To achieve this, it is a preferred feature of the present invention to provide easy to synthesize twofold negatively charged organic ligands that form charge neutral complexes together with divalent lanthanide cations.

The electroluminescent coordination compounds preferably show an increased sublimation yield such that large area OLED displays can be processed using low-cost sublimation or evaporation techniques.

The preparation of aza-crown ethers and cryptands is per se described in J. CHEM. SOC. PERKEN TRANS. 1, 1988, pages 3141 to 3147. It is stated that the simplicity of the described synthesis and the good yields obtained make the method suitable for large-scale preparation of aza-crown ethers and cryptands. No applications of the aza-crown ethers and cryptands are stated.

### Summary of the invention

In various aspects of this invention metal-organic coordination compounds and methods for synthesizing the same, mixtures containing the metal-organic coordination compounds, organic electronic devices comprising the metal-organic coordination compounds as well as methods for forming the same are provided.

The object is achieved by a metal-organic coordination compound as defined above and in claim 1.

In structure (1-1) **L₁, L₂** connected by two nitrogens form a basic cyclic group. The two nitrogens are further substituted by another two nitrogen containing groups with those nitrogens being finally linked with each other thereby forming a polycyclic organic ligand with the characteristic four nitrogen atoms as shown in formula (1-1). The length of **L₀**, **L₁, L₂** can be fine-tuned to optimally fit the size of a lanthanide cation, thereby providing high stabilization. However, to achieve stable complexes, the basis cyclic group formed by **L₁**, **L₂** and the two connecting nitrogens must be smaller than the 18-crown-6 motive and as such the combined shortest sequences of **L₁, L₂** must be less than 16 atoms. From ease of synthesis aspects preferably **R₂** - **R₅** in (1-1) are hydrogen. Preferably, in the ligand R₂ to R₅ are hydrogen, and the ligand is according to formula 1-2

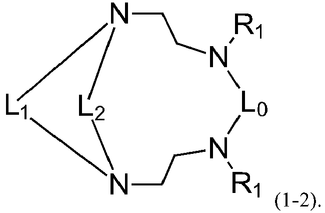

**R₁** is attached after the polycyclic ligand is formed and as such can be freely chosen. In particular with respect to deep blue electroluminescent application it is preferable to maintain an intrametallic transition and avoid an excited state energy transfer to the ligand. In the same context the excited state on the metal should not strongly polarize the organic ligand, as this again leads to efficiency loss of the emission process. Therefore, in preferred embodiments **L₀** in (1-1) is a substituted or non-substituted 2-9 membered linear aliphatic hydrocarbon chain in which one aliphatic chain member can be replaced by a hetero atom selected from N, B, P, O, S, preferably from N, and O. Even more preferably the hetero atom is O. Aliphatic as supposed to aromatic nature ensures sufficiently high triplet energy level of the ligand such that the excitation remains confined to the central metal cation. Hetero atoms ensure good complex formation by coordinating the central cation, yet preferably only N, and O and even more O are chemically hard and provide a fairly non-polarizable environment for the central cation.

In a further preferred embodiment **L₁,** and **L₂** in (1-1) are composed of linear chains of aliphatic ethylene fragments or ethylene and ethyleneoxy fragments, and the ligand has one of formulae 1-3-1 to 1-3-4.

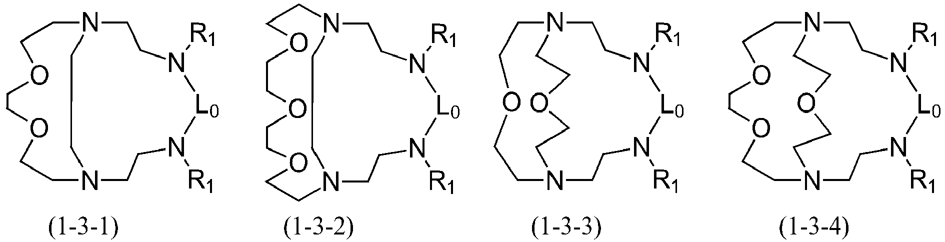

Again, this ensures a non-polarizable environment without accepting triplet energy levels on the ligand to ensure an excitation confined to the central cation. The cavity size of the basis ring, preferably made up of 4-5 hetero atoms together with corresponding members of carbon atoms preferably **L₁** and **L₂** with the connecting N atoms as shown in formulae 3-1-1 to 3-1-4 - with its combined shortest sequence of **L₁** + **L₂** of 8 to 15 atoms, preferably 9 to 14 atoms, more preferably 10 to 13 atoms, specifically 10 and 13 atoms, as in formulae 1-3-1 to 1-3-4 - perfectly fits the cation diameter of many lanthanides, and as such forms complexes of very high stability.

In another embodiment at least two atoms present in the shortest sequence of atoms of either **L₀**, **L₁,** or **L₂** are part of a benzene, pyridine, pyrrole, pyrazole, furan, carbazole, dibenzofurane or benzofuran ring structure. Here those aromatic building blocks are indeed more polarizable compared to oxygen. However, in particular for electroluminescence generation it may be highly desirable to have charge trapping or charge transporting functionality provided by the ligand. Yet still the ligand must have sufficiently high triplet energy level to ensure excitation confinement on the central metal cation. The named aromatic building blocks thus combine high triplet energy level above 2.7eV with reasonable charge transport or trapping properties.

In a preferred embodiment the lanthanide ion present in the metal-organic coordination compound is selected from Eu(II), Ce(III), and Yb(II), preferably from Eu(II), and Ce(III). Even more preferably the lanthanide is Eu(II). Eu(II), Ce(III), and Yb(II) all emit in the deep blue spectral region which is highly beneficial for electroluminescent applications. At the same time the transition of those three cations is of parity allowed d-f type, which ensures sufficiently short excited state lifetimes. The ground state of Yb(II) is a closed shell singlet state and during excitation unfavorable triplet states might be formed. This is not possible for Eu(II) and Cer(III). Yet for the latter, there is a single electron in the ground state, which leads to a relatively broad emission spectrum with two spectrally separated peaks, which is clearly less desirable than having a single peak pure emission spectrum. Finally, Eu(II) combines a deep blue and single peak emission spectrum, which is optically allowed and as such Eu(II) is the most preferred of the three lanthanide cations having blue d-f optical transitions.

In various embodiments the coordination compound comprises a polycyclic organic ligand according to formula (1-1) and at least one negatively charged anion that is not covalently linked to the polycyclic organic ligand. This is to ensure charge neutrality once the organic ligand is coordinating the positively charged cation. Preferably, the coordination compound is neutrally charged. If for example the polycyclic organic ligand is neutrally charged and the lanthanide is Eu(II), then two singly negatively charged anions or one doubly negatively anion is necessary for forming the overall neutrally charged coordination compound. This negatively charged counterion or anion can be any desired anion that fulfils the above-mentioned requirements. If the polycyclic organic ligand by virtue of a substituent (or two) is singly (or doubly) negatively charged, only one (or no) additional negatively charged anion is necessary to neutralize the Eu(II) coordination compound.

In various embodiments the coordination compound comprises a polycyclic organic ligand e.g. according to formula (1-1) and at least one negatively charged anion that is not covalently linked to the same, wherein the at least one negatively charged anion comprises of more than 2 atoms or has a molecular weight of more than 128g/mole. Such larger and/or more bulky anions ensure good stability against drift, if the coordination compound according to the invention is applied in a high electrical field environment, such as an OLED device.

Whilst any anions can be chosen for charge neutrality, of particular interest are those with sufficiently high triplet energy >2.7eV so that the excitation is confined to the metal-organic complex. Additionally, preferred anions do not significantly absorb in the visible spectral region and as such do not reabsorb any light generated by the central metal cation.

Generally preferred are coordination compounds comprising a polycyclic organic ligand e.g. according to formula (1-1) with **R₁** being H, deuterium, substituted or unsubstituted C₁₋₁₂-alkyl, aryl, aralkyl, heteroaralkyl, alkaryl, or with the two **R₁** being covalently linked with each other. Whilst **R₁** can be indeed chosen from a wide range of side groups those particular ones ensure a sufficiently high triplet level of the organic ligand and do not cause any polarization of the ligand in the presence of the excited central cation. In this context, R₁ is preferably H, deuterium, substituted or unsubstituted C₁₋₁₂-alkyl or substituted or unsubstituted C₆₋₁₈-aryl. If not stated otherwise, the expression "substituted or unsubstituted" refers to all meanings of a specific group that follow this expression.

In preferred embodiments, the ligand of the coordination compound has a structure according to formula (1-4): 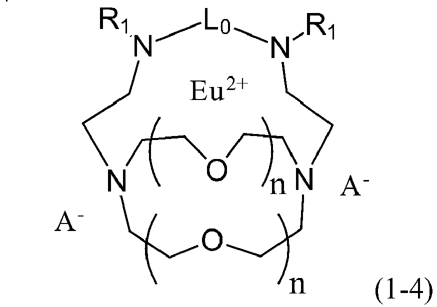 wherein
- **n** independently in each occurrence is 0, 1, 2, or 3, preferably 1 or 2, whilst the sum of both **n** is either 2 or 3 and
- **R₁** is H, deuterium, substituted or unsubstituted C₁₋₁₂-alkyl, aryl, aralkyl, heteroaralkyl, alkaryl, more preferably C₁₋₁₂-alkyl or C₆₋₁₈-aryl and wherein the two **R₁** can be covalently linked to form a cyclic group, and
- **L₀** is a substituted or non-substituted 2-9 membered linear aliphatic hydrocarbon chain in which one aliphatic chain member can be replaced by a hetero atom selected from N, B, P, O, S, preferably from N, and O. Even more preferably the hetero atom is O, and
- **A**⁻ is a singly negatively charged anion.

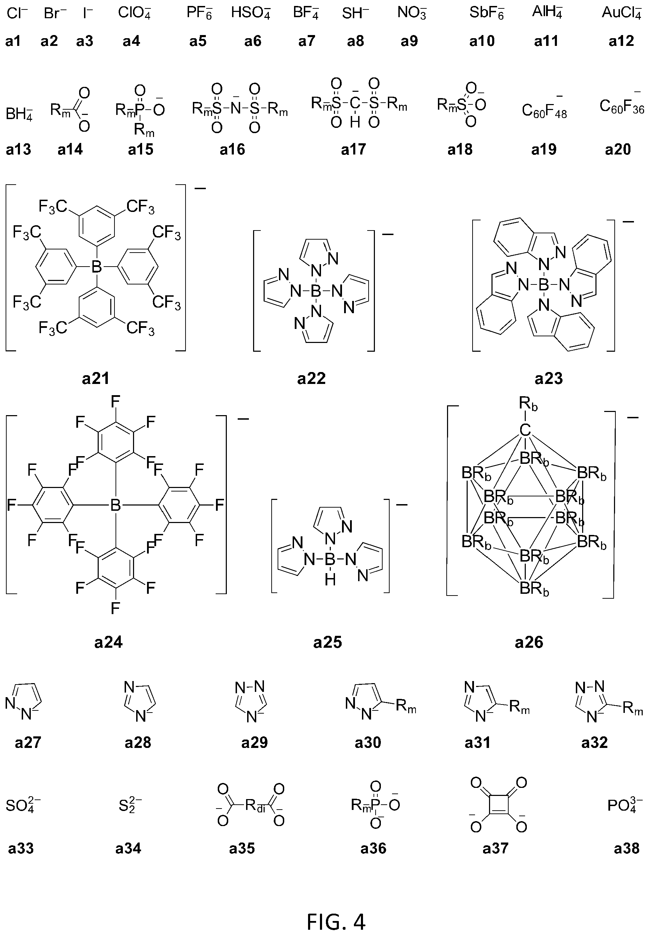

This particular embodiment of an organic polycyclic ligand is very well suited to coordinate Eu(II). Only weakly polarizable ethers with very high triplet level are used in the basic ring, ensuring excitation confinement on the central metal cation. Additionally, the basis crown ether, constituted by connecting **L₁,** and **L₂** via the two nitrogens has only 12 or 15 atoms including the two connecting nitrogens, ideally suited to coordinate Eu(II). Similar considerations or findings lead to the particular choices for **L₀** and **R₁**. This ligand is preferably combined with two identical singly charged anions, to allow for charge neutrality for the overall coordination compound. In preferred embodiments, for example for visible light generation, singly negatively charged anions with high triplet level above 2.7 eV and low absorption in the visible spectral region should be selected. Examples of suitable singly negatively charged anions are depicted in Figure 4. Thus, the ligand as shown in formula (1-4) ensures a good environment for Eu(II) leading to good stabilization of the central cation against oxidation and at the same time maintaining deep blue emission color with high emission efficiency.

In another embodiment the polycyclic ligand according to formula (1-1), (1-2), (1-3-1), (1-3-2), (1-3-3) and/or (1-3-4) and excluding the lanthanide, is at least singly negatively charged as such. In preferred embodiments no free anions are present in the coordination compound according to the invention. In other words, the ligand, for example according to formula (1-1), in combination with the at least one lanthanide cation, forms a complex of neutral charge and thus compensates the charge of the lanthanide. Such compounds without free (i.e. non-covalently bonded) negatively charged anions might be beneficial in electroluminescent device applications, where those very free anions may drift during operation in high electrical fields, leading to short device lifetimes.

In another embodiment the coordination compound comprises a polycyclic ligand according to formula (1-1), (1-2), (1-3-1), 1-3-2), (1-3-3) or (1-3-4) wherein **R₁** is selected from **b1** - **b23:** 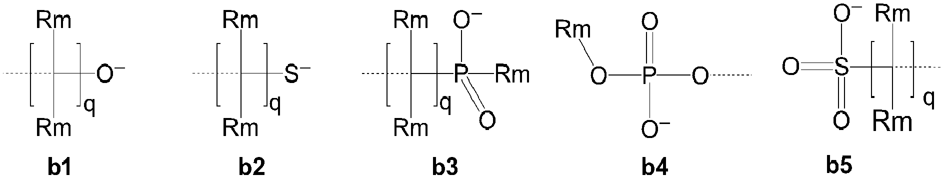 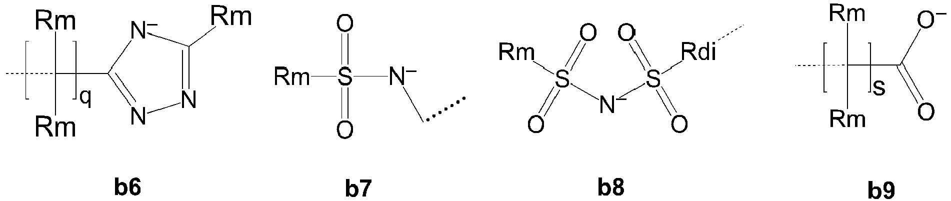 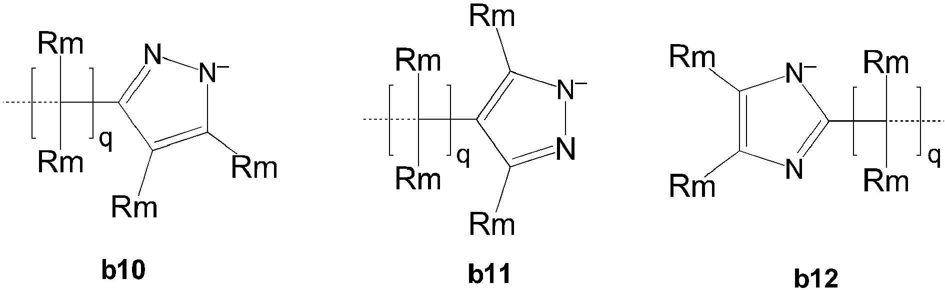 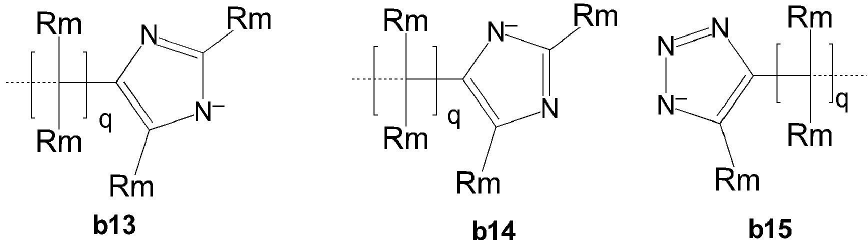 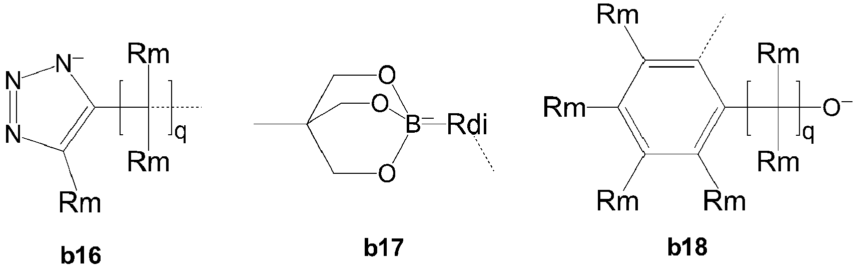 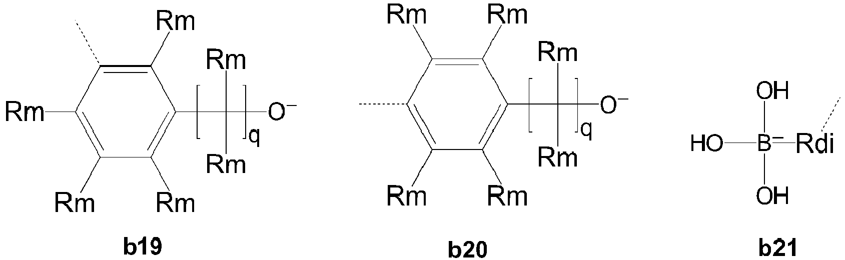 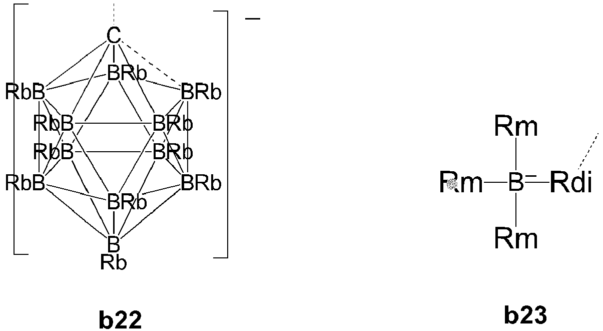 wherein
- the dashed lines indicate the linkage to the nitrogen atoms, in N-R₁ and
- **R_{b}** independently in each occurrence represents either hydrogen, halogen, or methyl
- **Rₘ** independently in each occurrence represents a monovalent group formed by removing a hydrogen atom from a substituted or non-substituted alkane, or arene, or heteroarene, and
- **R_{di}** represents a divalent group formed by removing two hydrogen atoms from a substituted or non-substituted alkane, or arene, or heteroarene, and
- **q** independently in each occurrence is 0, 1, 2, or 3 and
- **s** independently in each occurrence is 1, 2, or 3.

The depicted negatively charged side groups can all be chemically attached at the two nitrogen's **R₁** positions present in (1-1), (1-2), (1-3-1), 1-3-2), (1-3-3), (1-3-4). At the same time, they are robust and do not strongly polarize in the presence of the excited central cation. Again, sufficiently high triplet energy was ensured for the selection of those side groups.

In another set of embodiments the coordination compound and a second electrically neutral organic compound form a mixture, wherein the coordination compound is imbedded into the at least one second electrically neutral organic compound, wherein the second organic compound has a triplet energy higher than 2.5 eV, preferably higher than 2.6 eV and even more preferably higher than 2.7 eV and/or wherein the coordination compound has a higher hole affinity compared to the second organic compound. In applications of metal organic complexes, the material very often is used together with other organic materials. This embodiment ensures that the excited state of the emitting molecule is confined on the emitter. Here, if the triplet energy level of the surrounding material is too low, excitation transfer will cause low light generation efficiencies.

In another embodiment the coordination compound is covalently attached to a polymer with a molecular weight above 1000 g/mol, preferably to the polymer backbone.

In another embodiment, the coordination compound is used in an organic electronic device, comprising:
a first electrode;
a second electrode; and
an organic layer arranged such that it is electrically interposed between the first and second electrodes, wherein the organic layer comprises the
coordination compound, or the mixture or the polymer linked compound.

For many applications of the metal organic coordination compound a high thermal stability is needed, for example a high glass transition temperature may be desirable. For other applications, further functionalities might be needed, such as charge transport, or mechanical stability. Those latter functionalities could be provided using external polymeric materials. For processing for example from solution, or for better mixing, or thermal stability reasons it is helpful to covalently attach the metal organic coordination compound to a polymer.

Preferably, the coordination compound applied in the organic electronic device is deposited from the gas phase, in particular using an evaporation and/or sublimation and/or carrier gas process.

In various embodiments during the deposition from gas phase the coordination compound is blended with at least one second material, with the second material being present in the blend in an amount of between 1 and 99 vol%.

In another set of embodiments, the metal-organic coordination compound is used in an organic electronic device, preferably an organic light emitting device (OLED).

The invention also concerns the use of the metal-organic coordination compound in an organic electronic device, or as a dye or a contrast enhancement medium for magnetic resonance tomography, or of a polycyclic organic ligand as defined above for extracting three-valent actinides from radioactive waste water.

### Brief Description of the Drawings

In the drawings, like reference characters generally refer to the same parts throughout the different views. The drawings are not necessarily to scale, emphasis is instead generally being placed upon illustrating the principles of the invention. In the following description, various aspects of the invention are described with reference to the following drawings, in which:
**FIG. 1** exemplifies ligands according to the invention.
**FIG. 2** illustrate formulas of various possible side groups or substituents or parts of organyl groups of polycyclic organic ligands of the present invention;
**FIG. 3** illustrate formulas of various covalently bound anionic groups which can be present in the metal-organic complex according to the invention. The dotted line is the bond to the polycyclic organic ligand.
**FIG. 4** illustrate formulas of various non-covalently bound anionic groups which can be present in the metal-organic complex according to the invention.
**FIG. 5** exemplifies metal-organic complexes according to the invention
**FIG. 6** and **FIG. 7** illustrate schematic cross sections of organic electronic devices according to various aspects.
**FIG. 8****,** **9****,** **10****,** **11****,** **12****,** **13****,** **14****, and** **15** depict emission spectra of various complexes according to the invention.
**FIG. 16****,** **17****, and** **18** show OLED device characteristics using complexes according to the invention as active emitter.

The terms complex and coordination compound can be used interchangeably.

### Detailed Description

The following detailed description refers to the accompanying drawings that show, by way of illustration, specific details and aspects in which the invention may be practiced.

The word "exemplary" is used herein to mean "serving as an example, instance, or illustration". Any aspect or design described herein as "exemplary" is not necessarily to be construed as preferred or advantageous over other aspects or designs.

Various embodiments relate to metal organic compounds, including a lanthanide cation coordinated by a polycyclic organic ligand including at least four aliphatic amine groups and the applications of those compounds.

In this description, a coordination compound is taken to mean a compound where the central active metal is coordinated without a direct metal carbon bond.

In this description, an electroluminescent coordination compound is any material that is able to emit light upon electrical excitation, followed by recombination of electrons and holes. It shall be irrelevant in this context, whether the recombination of the electrons and holes takes place directly on the electroluminescent compound or first an excitation is formed on a different compound and subsequently transferred to the electroluminescent compound. Further, the electroluminescent coordination compound does not necessarily have to be used in an electronic device but, for example, may be used as a dye or a contrast enhancement medium for magnetic resonance tomography.

The lanthanide included in the coordination compound according to various embodiments may be any lanthanide cation, e.g. Yb2+, Eu2+, Sm2+, Ce4+, Ce3+, Gd3+, Eu3+, Er3+, or Yb3+, preferably Ce3+ or Eu2+, more preferably Eu2+.

The macrocyclic organic ligand according to various embodiments may be combined with actinides. For example, the polycyclic organic ligand may be size discriminating for three-valent actinides, allowing their extraction from radioactive wastewater. Yet for application in electroluminescent devices lanthanides are preferred vs radioactive actinides.

In this description, the arylene is a divalent organic fragment that is derived from an aromatic or heteroaromatic hydrocarbon (arene or heteroarene) by removing two hydrogen atoms from the aromatic or heteroaromatic hydrocarbon, preferably from different carbon and/or hetero atoms. One example is a (hetero) aromatic hydrocarbon that has had hydrogen atoms removed from two, preferably adjacent, hydrogen-bearing atoms (in case of aromatic hydrocarbon two carbon atoms, in case of heteroaromatic hydrocarbons two atoms selected from carbon and heteroatoms). An aromatic hydrocarbon or arene (or sometimes aryl hydrocarbon) is a hydrocarbon with sigma bonds and delocalized pi electrons between carbon atoms forming a circle. An alkylene is a divalent organic fragment that is derived from an alkyl group by removing one additional carbon atom.

The terms "halo," "halogen," and "halide" are used interchangeably and refer to fluorine, chlorine, bromine, and iodine.

The term "acyl" refers to a substituted carbonyl radical (C(O)-Rs).

The term "ester" refers to a substituted oxycarbonyl (-O-C(O)-Rs or-C(O)-O-Rs) radical.

The term "ether" refers to an -ORs radical.

The terms "sulfanyl" or "thio-ether" are used interchangeably and refer to a -SRs radical.

The term "sulfinyl" refers to a -S(O)-Rs radical.

The term "sulfonyl" refers to a -SO₂ -Rs radical.

The term "phosphino" refers to a -P(Rs)₃ radical, wherein each Rs can be same or different.

The term "silyl" refers to a -Si(Rs)₃ radical, wherein each Rs can be same or different.

In each of the above, Rs can be hydrogen or a substituent selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, and combination thereof. Preferred Rs are selected from the group consisting of alkyl, cycloalkyl, aryl, heteroaryl, and combination thereof.

The term "alkyl" refers to and includes both straight and branched chain alkyl radicals and can furthermore also include cycloalkyl radicals. Preferred alkyl groups are those containing from one to fifteen, preferably one to ten, more preferably one to five carbon atoms and includes methyl, ethyl, propyl, 1-methylethyl, butyl, 1-methylpropyl, 2-methylpropyl, pentyl, 1-methylbutyl, 2-methylbutyl, 3-methylbutyl, 1,1-dimethylpropyl, 1,2-dimethylpropyl, 2,2-dimethylpropyl, and the like. Additionally, the alkyl group is optionally substituted, e.g. by halogen or cycloalkyl.

The term "cycloalkyl radical" refers to and includes monocyclic, polycyclic, and spiro alkyl radicals. Preferred cycloalkyl groups are those containing 3 to 12, preferably 3 to 8, more preferably 3 to 6 ring carbon atoms and includes cyclopropyl, cyclopentyl, cyclohexyl, bicyclo[3.1.1]heptyl, spiro[4.5]decyl, spiro[5.5]undecyl, adamantyl, and the like. Additionally, the cycloalkyl group is optionally substituted, e.g. by halogen, alkyl or heteroalkyl.

The terms "heteroalkyl" or "heterocycloalkyl" refer to an alkyl or a cycloalkyl radical, respectively, having at least one carbon atom replaced by a heteroatom. Preferably the at least one heteroatom is selected from O, S, N, P, B, Si and Se, more preferably O, or B. Preferably 1 to 5, more preferably 1 to 3, most preferably 1 or 2 heteroatoms are present in the radical. The radical can be covalently linked with the remainder of the molecule via a carbon or heteroatom (e.g. N). Additionally, the heteroalkyl or heterocycloalkyl group is optionally substituted as indicated for alkyl and cycloalkyl.

The term "alkenyl" refers to and includes both straight and branched chain alkene radicals. Alkenyl groups are essentially alkyl groups with more than one carbon atom that include at least one carbon-carbon double bond in the alkyl chain. Cycloalkenyl groups are essentially cycloalkyl groups that include at least one carbon-carbon double bond in the cycloalkyl ring. The term "heteroalkenyl" as used herein refers to an alkenyl radical having at least one, preferably 1 to 5, more preferably 1 to 3, most preferably 1 or 2 carbon atoms replaced by a heteroatom. Preferably, the at least one heteroatom is selected from O, S, N, P, B, Si, and Se, more preferably O, S, or N. Preferred alkenyl/cycloalkenyl/heteroalkenyl groups are those containing two/three/one to fifteen carbon atoms. Additionally, the alkenyl, cycloalkenyl, or heteroalkenyl group is optionally substituted, as indicated above.

The terms "aralkyl" or "arylalkyl" are used interchangeably and refer to an alkyl group that is substituted with an aryl group. Additionally, the aralkyl group is optionally substituted, as indicated for alkyl and aryl.

The term "heterocyclic group" refers to and includes aromatic and non-aromatic cyclic radicals containing at least one, preferably 1 to 5, more preferably 1 to 3, most preferably 1 or 2 heteroatom. Preferably the at least one heteroatom is selected from O, S, N, P, B, Si, and Se, more preferably O, S, or N. Hetero-aromatic cyclic radicals may be used interchangeably with heteroaryl. Preferred hetero-non-aromatic cyclic groups are those containing 3 to 7 ring atoms which includes at least one hetero atom, and includes cyclic amines such as morpholino, piperidino, pyrrolidino, and the like, and cyclic ethers/thio-ethers, such as tetrahydrofuran, tetrahydropyran, tetrahydrothiophene, and the like. Additionally, the heterocyclic group may be optionally substituted, e.g. by halogen, alkyl or aryl. The heterocyclic group can be covalently linked with the remainder of the molecule via carbon and/or heteroatoms, preferably one carbon or nitrogen atom. The heterocyclic group can as well be linked with two carbon and/or heteroatoms with the remainder of the molecules. In the case the heterocyclic group can be part of a cyclic group.

The term "aryl" refers to and includes both single-ring aromatic hydrocarbyl groups and polycyclic aromatic ring systems. The polycyclic rings may have two or more rings in which two carbons are common to two adjoining rings (the rings are "fused") or wherein one carbon is common to two adjoining rings (e.g. biphenyl) wherein at least one of the rings is an aromatic hydrocarbyl group, e.g., the other rings can be cycloalkyls, cycloalkenyls, aryl, heterocycles, and/or heteroaryls. Preferred aryl groups are those containing three to eight aromatic carbon atoms. Especially preferred is an aryl group having six carbons. Suitable aryl groups include phenyl, and radialene. Most preferred is phenyl that can be substituted by non-aromatic groups. Additionally, the aryl group is optionally substituted, e.g. by halogen, alkyl, heteroalkyl, or deuterium. The aryl group is connected to the remainder of the ligand via one or two carbon atoms.

The term "heteroaryl" refers to and includes both single-ring aromatic groups and polycyclic aromatic ring systems that include at least one heteroatom. The heteroatoms include, but are not limited to and are preferably selected from O, S, N, P, B, Si, and Se. In many instances, O, S, or N are the preferred heteroatoms. Hetero-single ring aromatic systems are preferably single rings with 5 or 6 ring atoms, and the ring can have from one to five/six, preferably 1 to 3, more preferably 1 or 2 heteroatoms. The hetero-polycyclic ring systems can have two or more rings in which two atoms are common to two adjoining rings (the rings are "fused") or wherein one carbon is common to two adjoining rings (e.g. bipyridine) wherein at least one of the rings is a heteroaryl, e.g., the other rings can be cycloalkyls, cycloalkenyls. The hetero-polycyclic aromatic ring systems can have from 1 to 5, preferably 1 to 3, more preferably 1 or 2 heteroatoms per ring of the polycyclic aromatic ring system. Preferred heteroaryl groups are those containing three to thirty carbon atoms, preferably three to twenty carbon atoms, more preferably three to twelve carbon atoms. Suitable heteroaryl groups include dibenzothiophene, dibenzofuran, dibenzoselenophene, furan, thiophene, benzofuran, benzothiophene, benzoselenophene, carbazole, indolocarbazole, pyridylindole, pyrrolodipyridine, pyrazole, imidazole, triazole, oxazole, thiazole, oxadiazole, oxatriazole, dioxazole, thiadiazole, pyridine, pyrrole, pyrazole, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, phenoxazine, benzofuropyridine, furodipyridine, benzothienopyridine, thienodipyridine, benzoselenophenopyridine, and selenophenodipyridine, preferably pyridine, pyrrole, pyrazole, furan, carbazole, dibenzofurane or benzofuran groups, from which one hydrogen atom has been removed from a hydrogen-bearing carbon or heteroatom to form the covalent link to the remainder of the molecule. The heteroaryl group with two hydrogens removed can as well be linked with two carbon and/or heteroatoms with the remainder of the molecules, in which case the heteroaryl group can be part of a larger cyclic group. Additionally, the heteroaryl group is optionally substituted, e.g. by halogen, alkyl or aryl.

Generally, aryl and heteroaryl groups with triplet level > 2.5eV or more preferable >2.6eV or most preferably >2.7eV such as the groups derived from benzene, furan, dibenzofuran, dibenzoselenophene, carbazole, imidazole, pyridine, pyrazine, pyrimidine, triazine, and the respective aza-analogs of each thereof are of particular interest. There are many possible ways to probe the triplet energy level of an organic molecule. An easy way is to use suitable quantum mechanical calculations. A more direct way takes the onset of the phosphorescence spectrum, which is usually detected using gated spectroscopy, as pioneered by Romanovskii et al. "Phosphorescence of π-conjugated oligomers and polymers." Physical Review Letters 84.5 (2000): 1027.

The alkyl, cycloalkyl, heteroalkyl, heterocycloalkyl, alkenyl, cycloalkenyl, heteroalkenyl, alkynyl, aralkyl, heterocyclic group, aryl, and heteroaryl groups or residues, as used herein, are independently unsubstituted, or independently substituted, with one or more (general) substituents, preferably the substituents mentioned above.

Preferably, the (general) substituents are selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof with the number of carbon atoms and heteroatoms as defined above for the respective term. Furthermore, one or two substituents can be selected from polymer chains which can be covalently linked with the remainder of the molecule by a suitable organic spacer. Therefore, the cyclic organic ligand can be covalently linked with a polymer chain or a polymer backbone.

In some instances, the preferred general substituents are selected from the group consisting of deuterium, alkyl, cycloalkyl, heteroalkyl, alkoxy, aryloxy, amino, silyl, alkenyl, cycloalkenyl, heteroalkenyl, aryl, heteroaryl, nitrile, isonitrile, sulfanyl, and combinations thereof with the number of carbon atoms and heteroatoms as defined above for the respective term.

In yet other instances, the more preferred general substituents are selected from the group consisting of deuterium, alkyl, cycloalkyl, aryl, heteroaryl, and combinations thereof with the number of carbon atoms and heteroatoms as defined above for the respective term.

The terms "substituted" and "substitution" refer to a substituent other than H that is bonded to the relevant position, e.g., a carbon or nitrogen. For example, when R1 represents mono-substitution, then one R1 must be other than H (i.e., a substitution). Similarly, when R1 represents di-substitution, then two of R1 must be other than H. Similarly, when R1 represents no substitution, R1, for example, can be a hydrogen for available valencies of straight or branched chain or ring atoms, as in carbon atoms for benzene and the nitrogen atom in pyrrole, or simply represents nothing for ring atoms with fully filled valencies, e.g., the nitrogen atom in pyridine. The maximum number of substitutions possible in a straight or branched chain or ring structure will depend on the total number of available valencies in the ring atoms or number of hydrogen atoms that can be replaced. All residues and substituents are selected in a way that a chemically stable and accessible chemical group results.

As used herein, "combinations thereof" indicates that one or more members of the applicable list are combined to form a known or chemically stable arrangement that one of ordinary skill in the art can envision from the applicable list. For example, an alkyl and deuterium can be combined to form a partial or fully deuterated alkyl group; a halogen and alkyl can be combined to form a halogenated alkyl substituent; and a halogen, alkyl, and aryl can be combined to form a halogenated arylalkyl. In one instance, the term substitution includes a combination of two to four of the listed groups. In another instance, the term substitution includes a combination of two to three groups. In yet another instance, the term substitution includes a combination of two groups. Preferred combinations of substituent groups are those that contain up to fifty atoms that are not hydrogen or deuterium, or those which include up to forty atoms that are not hydrogen or deuterium, or those that include up to thirty atoms that are not hydrogen or deuterium counted for all substituents of a given molecule, or for the respective molecule in total. In many instances, a preferred combination of substituent groups will include up to twenty atoms that are not hydrogen or deuterium, counted for all substituents of a given molecule.

The "aza" designation in the fragments described herein, i.e. aza-cryptate, etc. means that one or more carbon atom or (other) heteroatom of a parent compound is replaced by a nitrogen atom, without any limitation. For example, in a crown ether -O- is replaced by -NH- to give the respective aza compound. One of ordinary skill in the art can readily envision other nitrogen analogs of the aza-derivatives, and all such analogs are intended to be encompassed by the terms as set forth herein.

As used herein, "deuterium" refers to an isotope of hydrogen. Deuterated compounds can be readily prepared using methods known in the art.

It is to be understood that when a molecular fragment is described as being a substituent or otherwise attached to another moiety, its name may be written as if it were a fragment (e.g. phenyl, phenylene, naphthyl, dibenzofuryl) or as if it were the whole molecule (e.g. benzene, naphthalene, dibenzofuran). As used herein, these different ways of designating a substituent or attached fragment are considered to be equivalent.

In some instances, a pair of adjacent or non-adjacent substituents or residues can be optionally joined (i.e. covalently linked with each other) or fused into a ring. The preferred ring formed therewith is a five-, six-, or seven-membered carbocyclic or heterocyclic ring, including both instances where the portion of the ring formed by the pair of substituents is saturated and where the portion of the ring formed by the pair of substituents is unsaturated. As used herein, "adjacent" means that the two substituents involved can be on the same ring next to each other, or on two neighboring rings having the two closest available substitutable positions, such as 2, 2' positions in a biphenyl, or 1, 8 position in a naphthalene, or 2,3-positions in a phenyl, or 1,2-positions in a piperidine, as long as they can form a stable fused ring system.

In this description, the term organyl refers to any chemically stable organic arrangement of atoms where one hydrogen atom has been removed such as to use this free valance to covalently link the organic group with another molecular entity. Thus, the term organyl encompasses the majority of the above defined organic groups e.g. fluorine, alkyl, cycloalkyl, heteroalkyl, alkoxy, aryloxy, amino, silyl, alkenyl, cycloalkenyl, heteroalkenyl, aryl, heteroaryl, nitrile, isonitrile, sulfanyl, and combinations thereof with the number of carbon atoms and heteroatoms as defined above for the respective term.

In all embodiments, the coordination compound contains a polycyclic aza cryptand according to the generic formula (1-1) 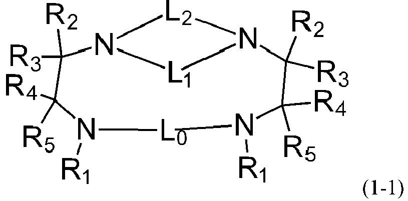 with
- **L₀**, **L₁, L₂** being independently each a divalent organic group formed by removing two hydrogen atoms from a substituted or non-substituted alkane, arylalkane, heteroarylalkane, arene, heteroarene, alkylarene, alkylheteroarene, dialkylarene or dialkylheteroarene, wherein alkane or alkyl can be interrupted by one or more oxygen or boron atoms, and with
- the sum of the number of atoms in the shortest sequences of atoms in **L₁,** and **L₂** between the two linkages of -L₁- and -L₂- and the remainder of the polycyclic ligand being less than 16 atoms, and
- **R₂** to **R₅** independently in each occurrence representing hydrogen, deuterium, halogen, or an organyl group, and
- **R₁** representing hydrogen, deuterium, or an organyl group.

In various embodiments, all **R₂** - **R₅** in formula (1-1) may be hydrogen such that the polycyclic organic ligand may described by the generic formula (1-2):

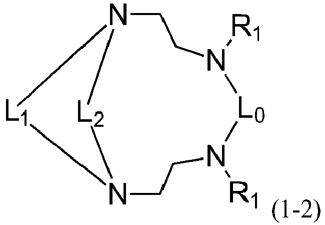

In various embodiments, the bridge **L₀** between the two adjacent nitrogens, **L₀**, in the generic structures of (1-1) and (1-2) above is a substituted or non-substituted 2-9 membered linear aliphatic hydrocarbon chain in which one aliphatic chain member can be replaced by a hetero atom selected from N, B, P, O, S, preferably from N and O, more preferably the hetero atom being O.

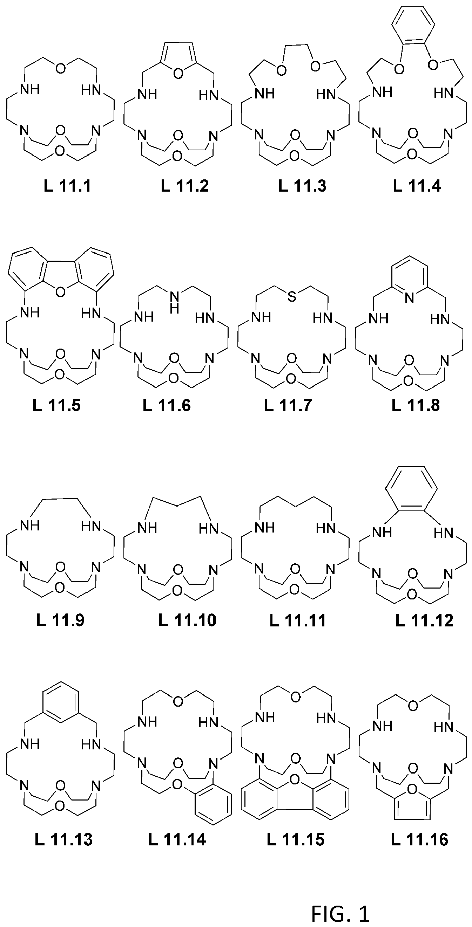

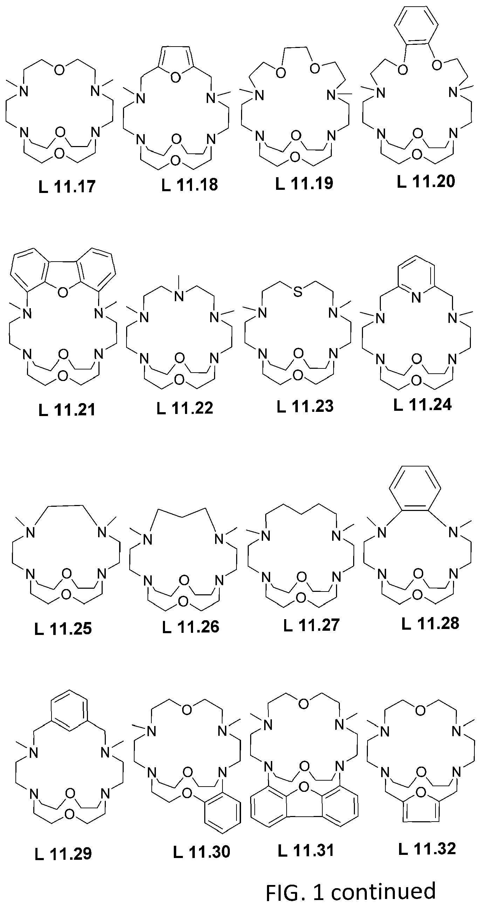

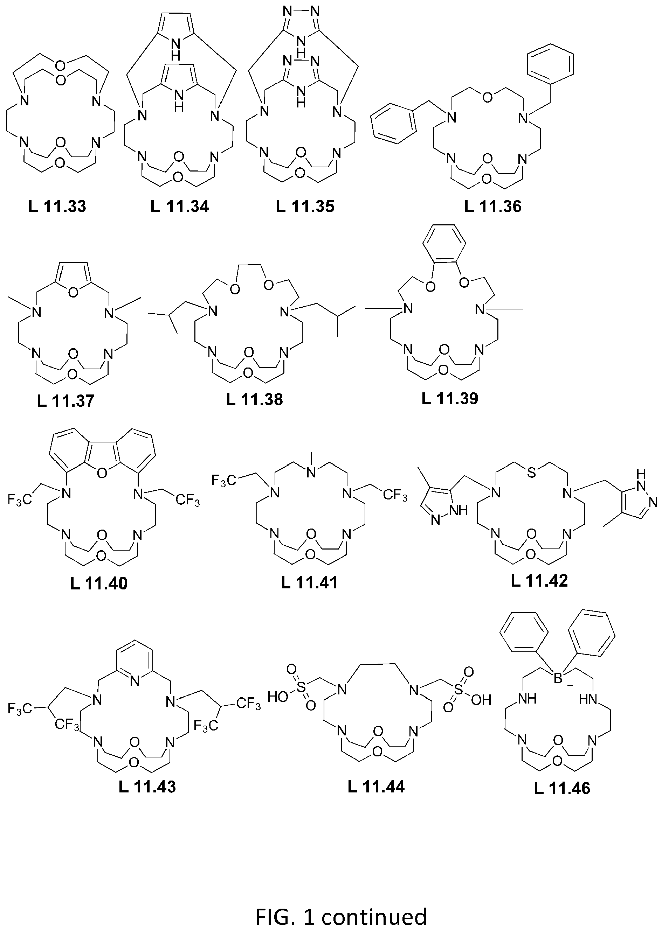

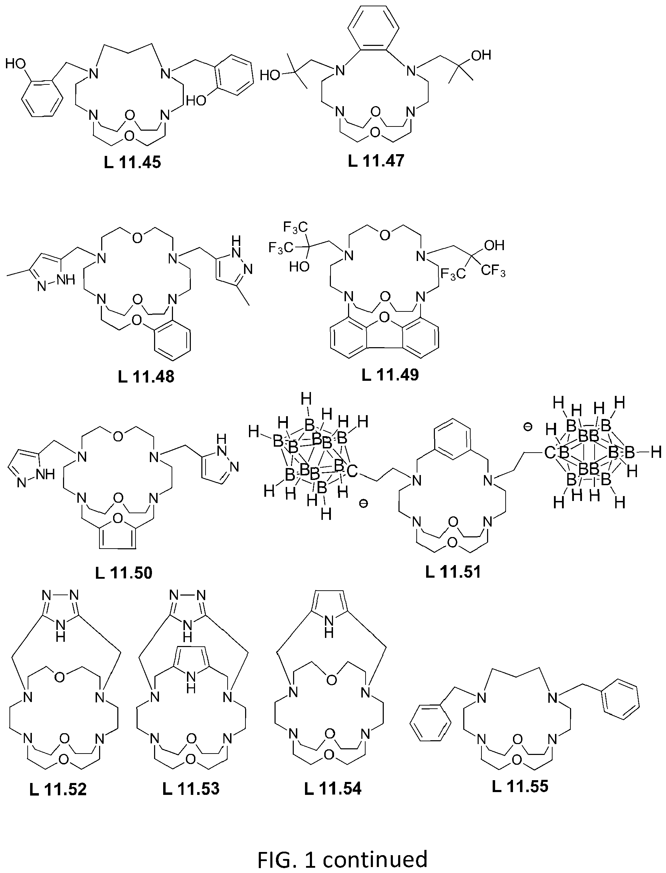

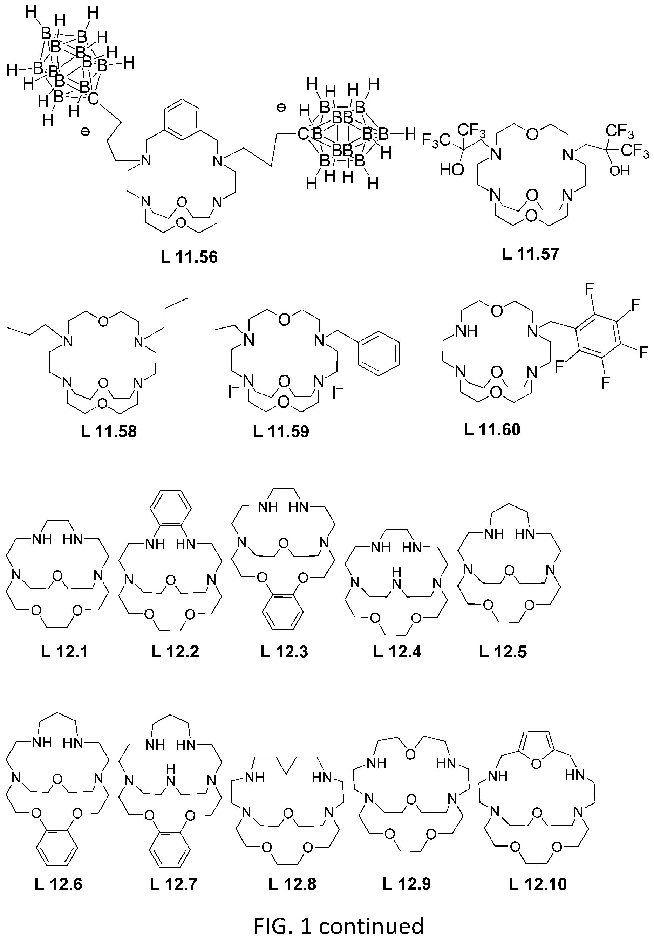

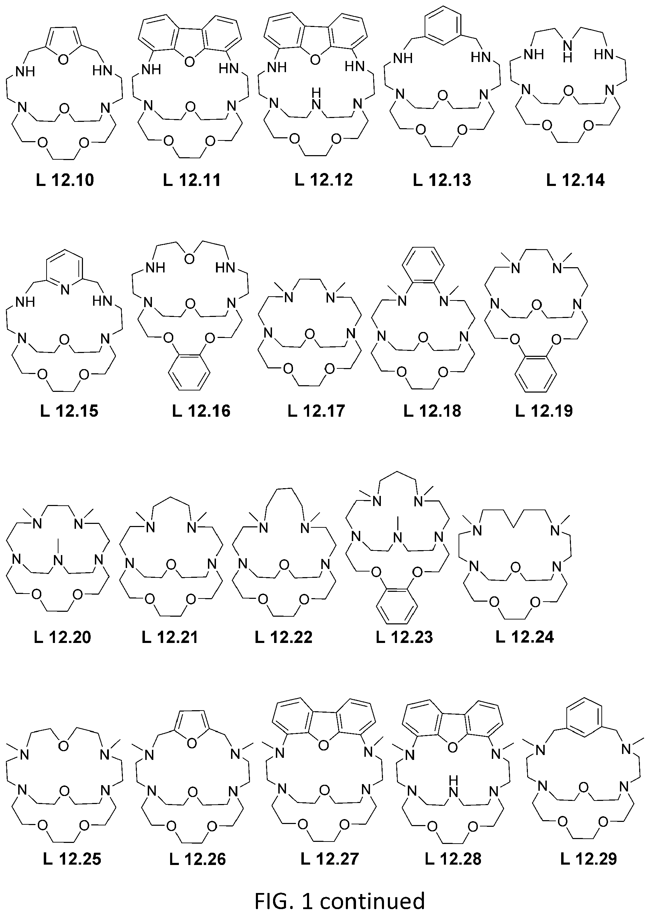

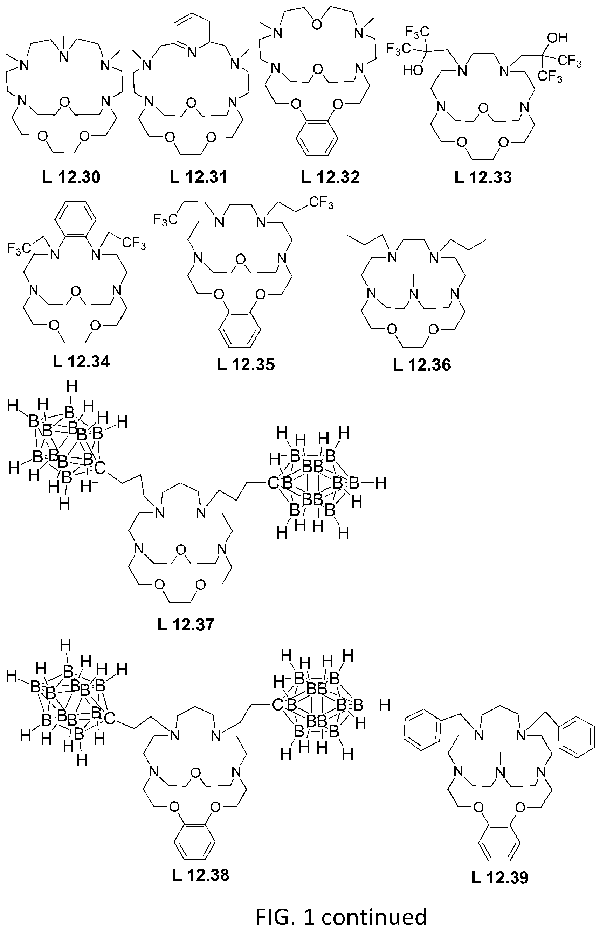

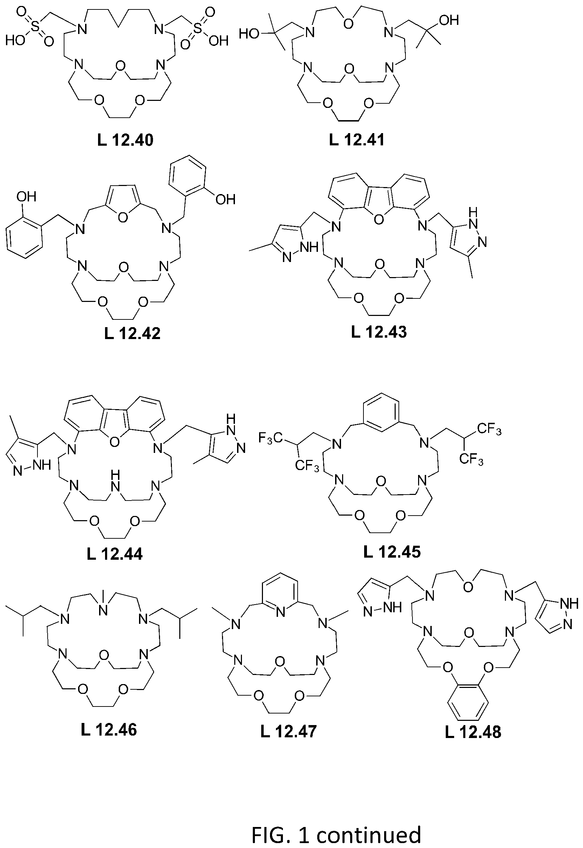

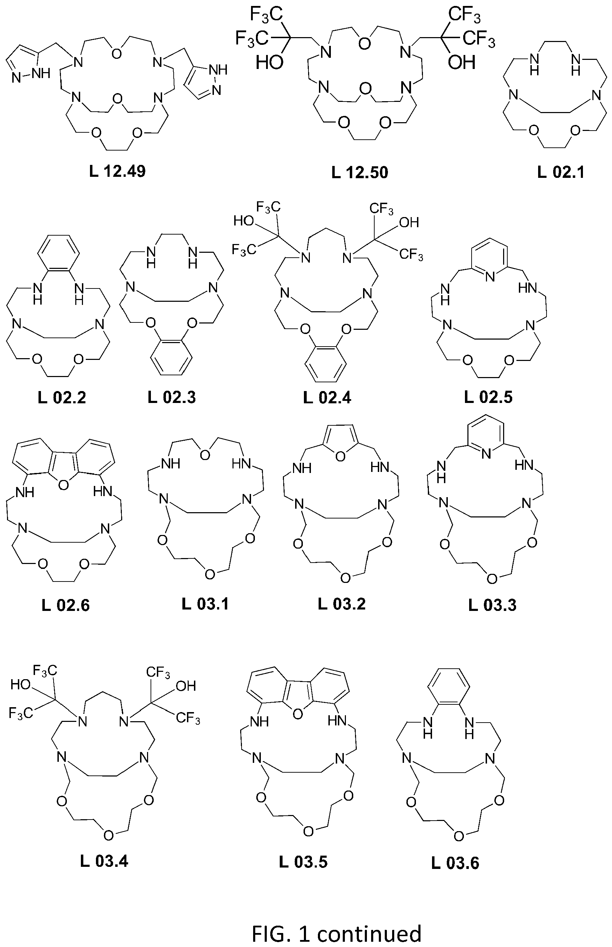

**FIG. 1** illustrates examples of the organic ligand according to various embodiments without limitation and represented by the generic formula (1-2) with linkers, **L₁, L₂**, and **L₀.**

In most examples depicted in **FIG. 1** the shortest sequence of the two linkers **L₁,** and **L₂** is constituted of one or more ethyl fragments or of one or more ethyl and one or more ethyloxy fragments. The only exceptions are L 12.4, 7, 12, 20, 23, 28, 36, 39, 44 in which **L₁** is based on diethyl amine fragments. The examples are grouped into four portions following the number of heteroatoms within **L₁,** and **L₂**, where labels starting with L 11 .XX represent examples in which both linkers **L₁**, and **L₂**, contain a single diethylether sequence only. Therefore, the shortest sequence, i.e. the shortest chain of atoms to link the two nitrogens, for both **L₁**, and **L₂**, is equal to 5 atoms. Thus, the sum of the two shortest sequences of **L₁,** and **L₂** is equal to 10 and as such being less than 16 atoms. Similar, labels starting with L 12.XX represent examples in which **L₁** comprises a single di ethyl amine or ether and **L₂** represents the corresponding diether or diamine organic fragment, with a combined shortest sequence of atoms of 13. In examples labeled with L 02.XX **L₁** comprises an ethyl fragment, whilst **L₂** is a diether organic fragment - the sum of the shortest sequences being 10. Finally, in examples labeled with L 03.XX **L₁** comprises an ethyl fragment, whilst **L₂** is a triether organic fragment - the sum of the shortest sequences being 13.

Therefore, the examples depicted in **FIG. 1****,** wherein the shortest sequence of **L₁,** and **L₂** are comprised of ethyl or diethylether fragments, can be summarized with the four generic formulas shown in 1-3-1 to 1-3-4.

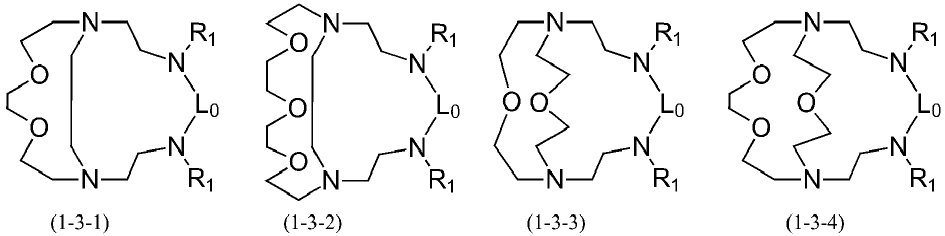

One, two, or three oxygens present in these formulae may be substituted by nitrogens, i.e. N-R₆, with the free valence R₆ being hydrogen, or deuterium, or an organyl group, therefore arriving at the generic aza-analogons, partly based on diethylamine. In **FIG. 1****,** L 11.01 until L 11.13 represent examples of **L₁**, and **L₂**, falling under the second structure from the right in (1-3-3), whilst L 12.01 until L 12.15 correspond to the rightmost structure (1-3-4). (1-3-3) and (1-3-4) are preferred structures of the ligand.

In various embodiements, two atoms present in the shortest sequence of atoms of either **L₀**, **L₁,** or **L₂** are part of a benzene, pyridine, pyrrole, pyrazole, furan, carbazole, dibenzofurane or benzofuran ring structure. For example, in L 11.14, L 11.30, L 11.48, L 12.23, L 12.35 a benzene ring is fused into the shortest sequence of **L₁.** Similar, in L 11.49 and L 11.50 furan and dibenzofurane are part of the shortest sequence of **L₁**. "Shortest sequence" shall always be the shortest sequence (and number) of atoms that link the two nitrogens. By example of L 11.14 unit! L 11.16, the sortest sequence of **L₁**, and **L₂** is always equal to 5. For example, the benzene ring in L 11.14 is "fused" onto the shortest sequence of the linker atom sequence.

The two nitrogens together with the two covalently bonded linkers **L₁**, and **L₂** form a basic cyclic di-aza-crown ether. According to the generic formula (1-1) both nitrogens forming a cycle with L₁ and L₂ are further substituted in a characteristic manner thereby linking with further two nitrogens via a spacer group -(C(R₂R₃)-C(R₄R₅)-. The polycyclic ligand according to formula (1-1) is completed by connecting those further two nitrogens with the linker **L₀.** A wide range of linkers **L₀** is chemically easely acessible, preferably chosen from substituted or non-substituted alkane, arene, or heteroarene. In order to ensure stable complex formation as part of the metal-organic coordination compound, the preferred linker has a shortest sequence of not more than 8 atoms. For similar reasons it may comprise one or more hetero atoms able to complex and therefore stabilize the metal cation to be coordinated. For example, in **FIG. 1** the linkers **L₀** in L 11.3 and L 11.4 comprise of a shortest sequence of 8 atoms comprising two coordinating oxygens, whilst in L 11.9 the linker **L₀** is a simple two-atom 1,2-ethylene bridge without any hetero atoms. **L₀** is by no means restricted to simple alkylene or heteroalkylene chains. In various embodiements **L₀** preferably contains alkyl, heteroalkyl, aryl or heteroaryl groups as substituents or part of the ring structure. In various embodiments the metal-organic coordination compound comprising the ligand according to formula (1-1) is used to generate deep blue light with high emission yield. This is best achieved, if the excitation is confined on the central metal, thereby avoiding significant formation of charge transfer states or energy transfer between the central metal and the coordinating ligand according to formula (1-1). To ensure energy confinement on the metal central atom, the ligand should have a triplet energy higher than the emission energy of the intrametallic transition. Consequently, the triplet energy of the ligand must be at least >2.5eV, preferably >2.6eV and even preferably >2.7eV. Triplet energies are measured e.g. as described in Romanovskii, Yu V., et al. "Phosphorescence of π-conjugated oligomers and polymers." Physical Review Letters 84.5 (2000): 1027. Consequently, the buildings blocks of the polycyclic organic ligand according to formula (1-1) are preferably either non-aromatic (aliphatic) or comprises of aromatic building blocks containing at most 8 aromatic carbon or hetero atoms, whereby various aromatic building blocks are being separated from each other by at least one nonaromatic carbon or heteroatom. For illustration, examples according to the invention are shown in **FIG. 1****.** Here, L 11.1, L 11.7 and L11.3 are fully aliphatic. On the other hand L 11.2, L 11.4, L 11.5, L11.8 comprise aromatic building blocks in particular as part of **L₀,** namely furane, benzene, dibenzofurane, and pyridine. Furane, benzene, and pyridine contain less than 8 aromatic carbons or heteroatoms. Dibenzofurane can be considered as two benzene rings linked by non-aromatic hetero or carbon atoms according to the definition above and thus ensuring sufficiently high triplet energy of the ligand system.

Specifically preferred embodiments for the polycyclic organic ligands are as follows: L₀ has a sum of the shortest sequence of atoms in L₀ (between the two linkages of - L₀- and the remainder of the polycyclic ligand) of preferably 2 to 9 atoms, more preferably 2 to 7 atoms, most preferably 2 to 5 atoms. In particular, L₀ is -C₂H₄-, - C₃H₆- or -C₂H₄-O-C₂H₄-.

L₁ preferably is -C₂H₄- or -C₂H₄-O-C₂H₄-, more preferably -C₂H₄-O-C₂H₄-.

L₂ preferably is -C₂H₄-O-C₂H₄-O-C₂H₄- or -C₂H₄-O-C₂H₄-.

R₂ to R₅ preferably are hydrogen atoms. R₁ preferably is hydrogen, methyl, benzyl, - CH₂-C(CF₃)₂OH or the group depicted in Figure 1, L 11.42.

In Figure 1, different types of core ring structures can be identified. Type 1 is shown as L 11.17 with two methyl substituents. Type 2 is shown in L 12.1 and L 12.5 with an ethylene bridge or propylene bridge on top. Type 3 is shown in L 12.9.

Substituted type 2 can be seen in L 12.17 and L 12.21, substituted type 3 in L 12.25.

With regard to formulae (1-3-1) to (1-3-4), in Figure 1 L 11.1, L 11.17, L 11.36, L 11.57 illustrate formula (1-3-3). L 12.1, L 12.5, L 12.9, L 12.17, L 12.21, L 12.25, L 12.49 and L 12.50 illustrate formula (1-3-4). These formulae are particularly preferred.

According to one preferred embodiment, L₀, L₁, L₂ independently have the formulae - (CH₂-CH₂-O)ₓ-C₂H₂- with x being 1, 2, 3 or 4, more preferably 1, 2 or 3, most preferably 1 or 2.

L₀ can also be -(CH₂-)_{y}, with y being 2, 3, 4, 5 or 6, more preferably 2, 3 or 4, most preferably 2 or 3.

According to one embodiment, a group CHR being in the shortest sequence of atoms in L₀, L₁ or L₂ between the two linkages of -L₀-, -L₁- and -L₂- and the remainder of the polycyclic ligand, may be exchanged for a group NR or BR, if the neighboring atoms are not hetero atoms. R can be as defined for R₁.

Preferably, substituents are C1-4-alkyl or halogen, more preferably methyl or ethyl, if they are different from hydrogen.

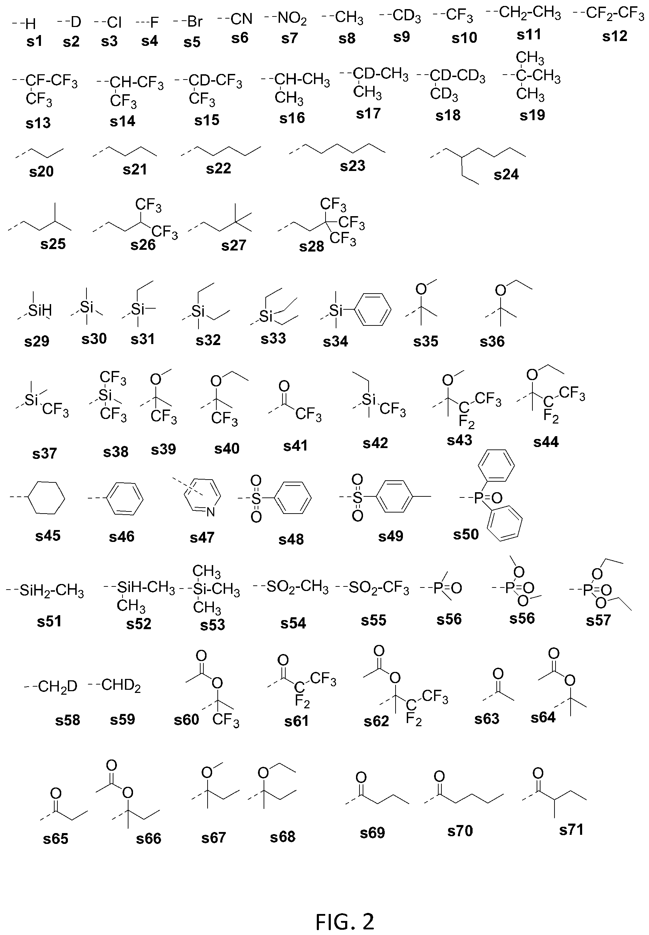

The coordination compound according to various embodiments may contain a ligand according to formula (1-1) with a plurality of substituents at positions **R₁** - **R₅**, which are not equal to hydrogen. Preferably 0 to 10, more preferably 0, 2 or 4 and even more preferably 0 or 2 of these substituents can be present. Those substituents may be any chemical fragment that can be covalently attached in accordance with the formula (1-1). Examples of suitable substituents s2 to s71 are illustrated in **FIG. 2** wherein the dashed line indicates the covalent link of the substituent with the main part of the ligand. In the generic formula of the ligand (1-1), **R₂** - **R₅** independently in each occurrence represent hydrogen, or deuterium, or halogen, or an organyl group. Therefore a wide variety of substitution patterns can be considered and may be preferable in various embodiments of this invention. In **FIG. 1** all **R₂** - **R₅** are invariably set to hydrogen, for sake of clarity. This, however, shall not limit the general scope of the invention, as any chemical fragment that can be covalently attached is in the scope of the invention. Examples of charge neutral substituents are s2 to s71 illustrated in **FIG. 2** whereby a dashed line indicates the preferred covalent link of the shown molecular fragment to the carbon atoms of the polycyclic ligand according to formula (1-1). Those fragments are meant as illustration for the scope of the invention only. Many more side groups may be chosen, depending on the final application. For example, linear or branched alkane side chains may be chosen, if a good solubility in non-polar solvents is needed. For other applications, coordination compounds with high thermal stability, for example suitable for evaporation or sublimation, may be needed. In this case, small and light weight side groups **R₂** - **R₅** are preferably employed, i.e organyl fragments with individually less than 20 atoms and or individually a molecular weight of less than 100g/mol. Similar, halogenated, particularly fluorinated, side groups may improve volatility and/or stability of the coordination compound according to the invention. In case the intended application of the coordination compound of the invention is light generation, for example by electroluminescence, the ligand ideally has to be chosen such that the excitation of the coordination compound is confined to the central lanthanide cation. In this way an intrametallic transition with highest efficiency is obtained. With respect to the choice of **R₂** - **R₅**, preferred are side groups that are not easily polarized and that have a sufficiently high triplet level. Low polarization is ensured for aliphatic or heteroaliphatic side groups **R₂** - **R₅** or using small aryl- or heteroaryl systems with less than 8 conjugated atoms or heteroatoms. Sufficiently high triplet energy obviously depends on the emission color of the complex. For example, if the intended emission color is in the near infrared area, for example by using Er3+ as the central lanthanide, side groups comprising of extended aryl, or heteroaryl organyl groups, such as phenanthroline, may still offer sufficiently high triplet level to ensure confinement of the excitation on the metal cation. On the other hand, if the coordination compound is intended to emit in the visible, and in particular in the deep blue spectral region, then side groups with a triplet energy >2.5 eV, or preferably >2.7 eV should be used. Sufficiently high triplet energy >2.7eV is generally observed for organyl groups comprising of aliphatic or heteroaliphatic fragments or for fragments comprising only small aryl- or heteroaryl systems with less than 8 conjugated atoms or heteroatoms, which thus should preferably be employed at the positions **R₂** - **R₅** if a deep blue electroluminescent emitter is the intended use of the coordination compound according to the invention. Examples of suitable side groups with respect to their triplet energy level are s2 to s71 illustrated in **FIG. 2****. R₁** is the third valency of the two nitrogen atoms that connect to the linker **L₀** in the generic formula (1-1). Form a synthesis point of view, this valency can very easily be substituted at a late stage of the building of the organic ligand, allowing for a rich body of options that are easy to implement. In various embodiments, **R₁** may be used to link the ligand system to a separate organic compound such as a polymer or dendrimer. Alternatively, **R₁** may be chosen specifically such as to bind the whole metal organic coordination complex to a surface, such as gold. In the simplest case, the nitrogen valencies are non substituted, i.e. both **R₁** are hydrogen. In **FIG. 1** this situation is for example realized in example L 11.1 until L 11.16. In examples L 11.17 until L 11.32 methyl groups are chosen for **R₁**. In various examples of the invention it may be prefered to link the two **R_{1S}** of the ligand represented by formula (1-1) with each other, which may be beneficial for the shielding or generally for the stability of the complex. Examples L 11.33 until L 11.35 in **FIG. 1** represent the situation of both **R_{1S}** linked together. In those particular examples the linker formed by connecting the two **R_{1S}** is chosen to be equal to **L₀.** Yet, it is understood that in the general scope of the invention those two linkers can be different, which is for example the situation in L11.54, L11.59, and L11.60. Obviously, the linkers depicted for example in L 11.33 until L 11.35 in **FIG. 1** cannot formally be broken in two identical halves to give to identical **R_{1S}** as represented in the generic formula (1-1). Nevertheless, this representation is explicitly included in the scope of the invention.

In various embodiments, **R₁** are charge neutral substituents with 1 to 40 atoms being either non-aromatic (aliphatic) or comprise aromatic building blocks containing at most 8 aromatic carbon or heteroatoms, whereby various aromatic building blocks are being separated from each other by at least one non-aromatic (alipatic) carbon or heteroatom, which ensures sufficiently high triplet energy level to confine a blue excitation on the central metal. Suitable substituents may be chosen from s2, and s6 to s71 illustrated in **FIG. 2** whereby a dashed line indicates the preferred covalent link of the shown molecular fragment to the nitrogen atoms of the polycyclic ligand according to formula (1-1). The fragments depicted in **FIG. 2** are only meant as examples and should not limit the general scope of the invention.

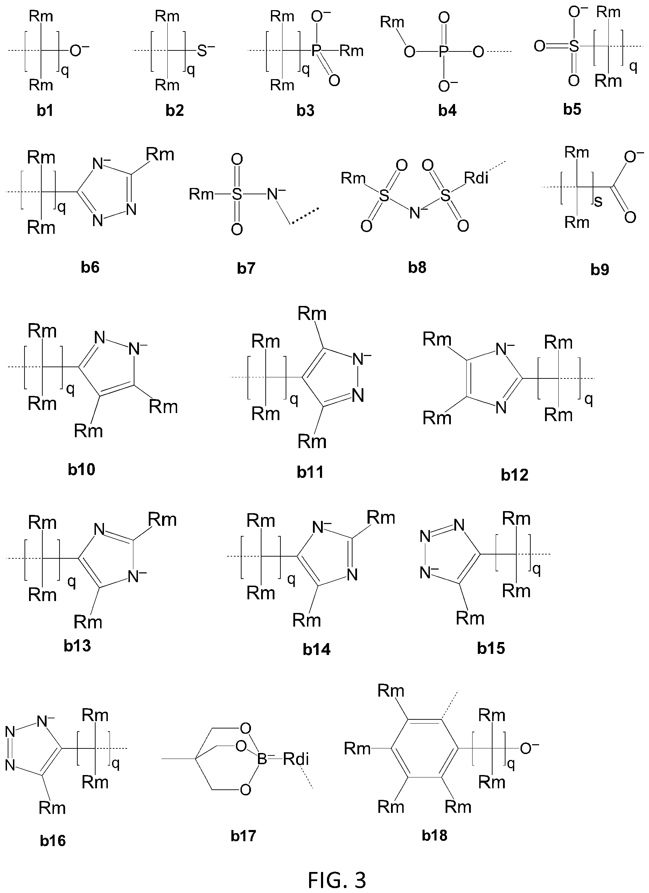

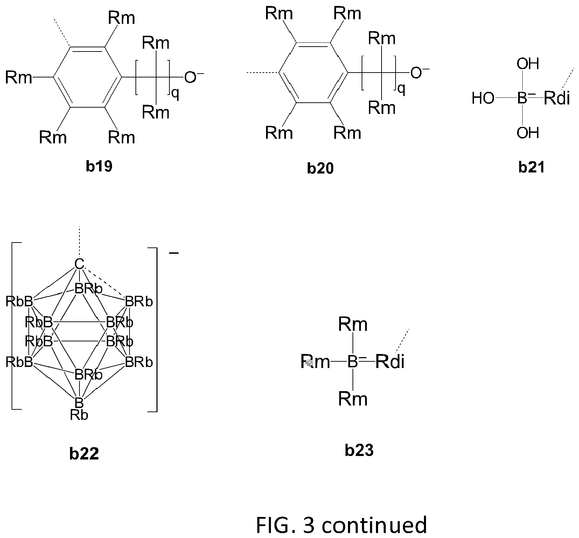

In various embodiments, the polycyclic organic ligand according to formula (1-1) that coordinates to a lanthanide cation within the coordination compound according to invention may itself already be single or multiple negatively charged. In various embodiments, suitable anionic groups are covalently attached to the polycyclic organic ligand according to formula (1-1) at positions **R₁** - **R₅**. Examples of such monoanionic substitutions are illustrated in b1-b59 in **FIG. 3**
wherein
- the dashed lines indicate the linkage to the nitrogen or carbon atoms, and
- **R_{b}** independently in each occurrence represents either hydrogen, halogen, or methyl
- **Rₘ** independently in each occurrence represents a monovalent group formed by removing a hydrogen atom from a substituted or non-substituted alkane, or arene, or heteroarene, and
- **R_{di}** represents a divalent group formed by removing two hydrogen atoms from a substituted or non-substituted alkane, or arene, or heteroarene, and
- **q** independently in each occurrence is 0, 1, 2, or 3 and
- **s** independently in each occurrence is 1, 2, or 3.

Instead of covalently attaching anionic groups, the polycyclic organic ligand may as well comprise negatively charged hetero atoms, such as borates, or negatively charged aryl groups, such as pyrazolyl, as part of the scaffold of the polycyclic organic ligand. For example this can be achieved by deprotonation of the aromatic building blocks depicted as L 11.34, L 11.35, L 12.41, L 12.33, and L 12.40 and so on in **FIG. 1****.** Alternative, formally negatively charged closocarborane clusters may be attached to the organic ligand, compare examples L 11.37, and L 11.38 for illustration purpose.

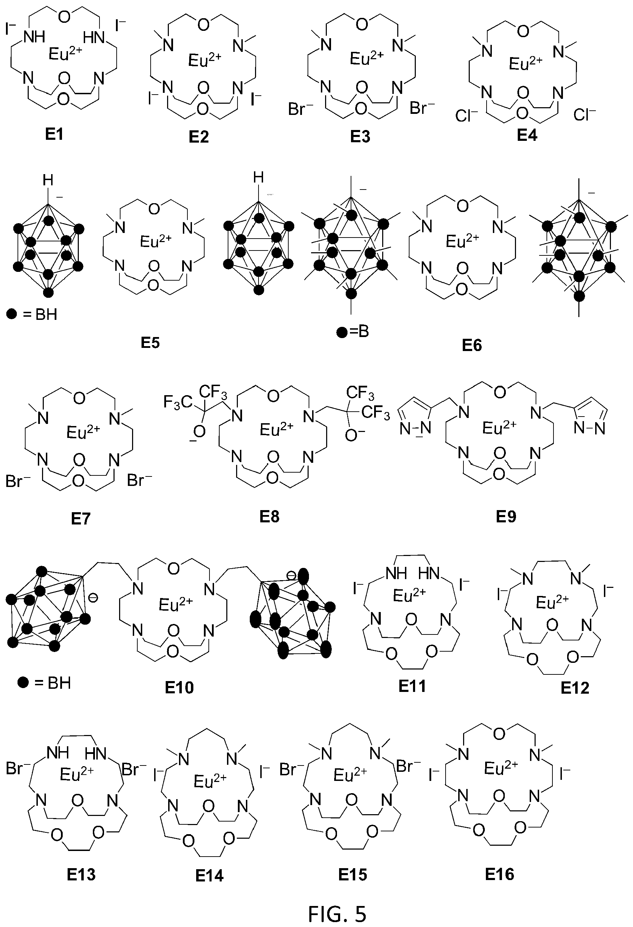

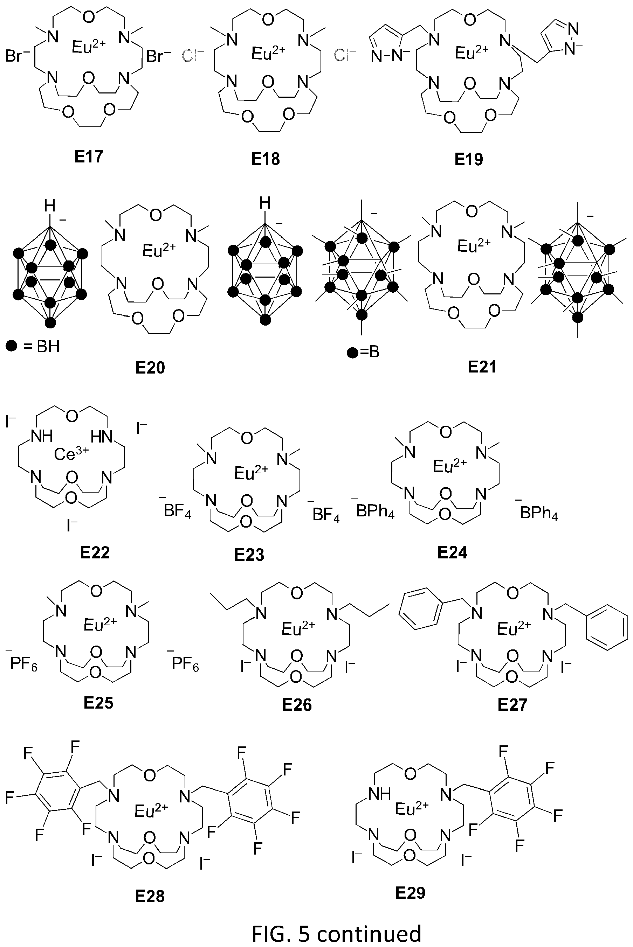

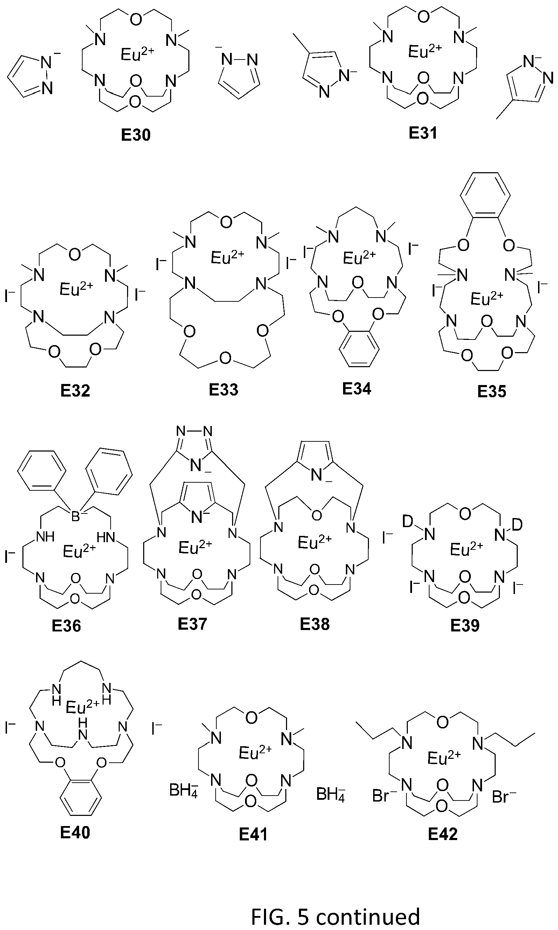

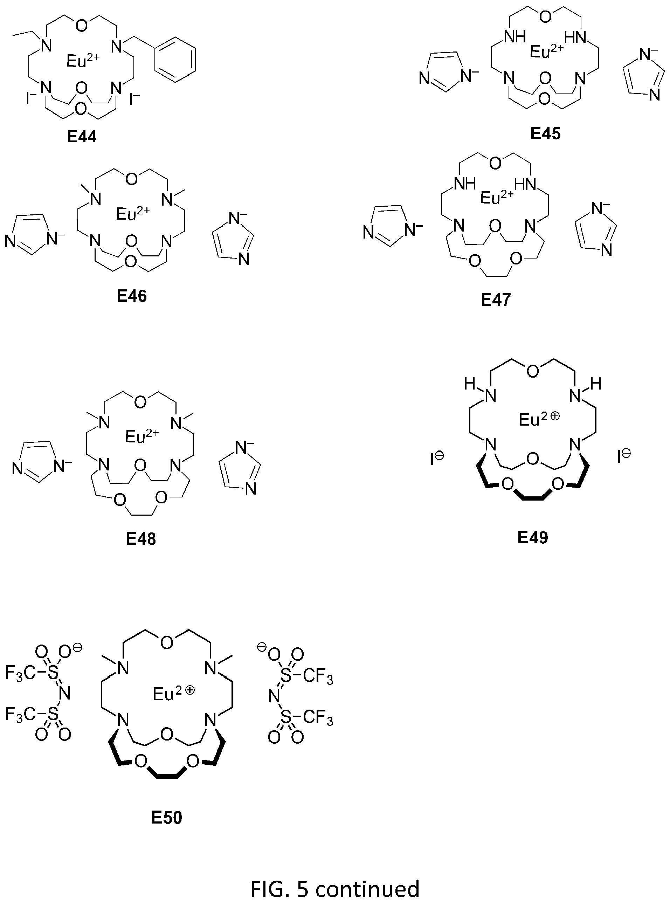

In preferred embodiements for the coordination compounds of the present invention, the sum of the negative charges of the covalently attached anionic groups matches the charge of the central lanthanide cation and thus the ligand, by coordinating the central lanthanide cation, forms a complex of neutral charge. In such configuration no unbound negatively charged anions are present, therefore anion drift in device configuration under applied electric fields is elegantly circumvented. For example if the central lanthanide is chosen to be divalent Europium, Eu2+, then the ligand may contain exaclty two singly negatively charged anionic groups or exatly one twofold negatively charged anionic group. Examples E8, E9, and E10 in **FIG. 5** serve for illustration for the case of two singly negatively charged anionic groups combined with divalent Europium as central cation. There may be three singly negatively charged anionic groups present, in case the central lanthanide is Ce3+. Alternatively, such a charge neutral metal organic Ce(lll) compound may contain one singly combined with one twofold negatively charged anionic group or alternatively only one threefold negatively charged anionic group is for example covalently attached at positions **R₁** - **R₅** to the ligand according to formula (1-1).

The negatively charged anionic side groups depicted in **Fig. 3** shall not limit the scope of the invention. In various embodiments, anionic groups covalently linked to the organic polycyclic ligand may be twofold or even higher charged, for example by attaching sulfate, phosphonate, or chromate anionic groups. Similarly, the polycyclic organic ligand may be negatively charged higher than 3. In such a case, a charge neutral compound may be obtained by incorporation of additional suitable organic or inorganic cations, such as ammonia, alkali, alkaline earth metal cations or similar into the coordination compound according to invention.

The optional negative charge present in the ligand may as well be substantially localized on a particular heteroatom that is part of the scaffold of the organic ligand according to the invention that is generically represented by formula (1-1). For example in **Fig. 1** L 11.46 a negatively charged borate is part of the linker **L₀,** illustrating a singly negatively charged ligand according to formula (1-1).

In various embodiments, the coordination compound may include at least one negatively charged anion, which is not covalently bound to the polycyclic organic ligand according to formula (1-1). For illustration some suitable anions are depicted in **Fig. 4****,** wherein
- **R_{b}** independently in each occurrence represents either hydrogen, halogene, or methyl, and
- **Rₘ** independently in each occurrence represents a monovalent group formed by removing a hydrogen atom from a substituted or non-substituted alkane, or arene, or heteroarene, and
- **R_{di}** represents a divalent group formed by removing two hydrogen atoms from a substituted or non-substituted alkane, or arene, or heteroarene.
- R_{b}, Rₘ, R_{di} can also have the same meaning as for FIG.3 above.

The negatively charged anion may be a small inorganic anion such as, but not limited to a1 to a13 in **FIG. 4****.** When an external field is applied to a layer comprising the coordination compound according to invention, for example in operative OLED device configuration, anions may drift towards oppositely charged electrodes especially when using small and non-bulky anions. Such behavior may define a so-called light emitting chemical cell.

A light emitting chemical cell may be an embodiment of an OLED in this description. Drift of charged species within a device including the lanthanide coordination compound having an aza-cryptand ligand according to formula (1-1) may lead to very low driving voltages, which may assist to facilitate very good power efficiencies. This may be desirable for some applications, such as general illumination or signage. Yet, for other applications that require fast response times, for example flat panel displays, such ion drift may lead to time dependent OLED characteristics, which may be difficult to control, and may not be desirable as such. Therefore, the choice of the anions may depend strongly on the application.

In various embodiments, the coordination compound may contain comparably large and bulky organic anions. Such anions may be employed if ion drift is not desired. Examples of such anions include fluorinated or non-fluorinated fullerene (C60-, C60F36-, C60F48-) fluorinated aryl, carboranes, and borates, Examples a14 to a32 thereof are illustrated in **FIG. 4** but are not limited thereto.

The negatively charged anion may include more than one atom, preferably more than three atoms, and/or can have a molecular weight of at least 128 g/mol. Such larger anions are generally more bulky or heavier and as such are better suited for applications where drift of anions is not desired.

In various embodiments, anions without substantial absorption in the visible spectral region may be used. In case there are two or three non-covalently bound anions, they may be the same or of different type.

In various embodiments, anions comprising bor, known as borates, or boron clusters, known as carboranes, may be used.

In various embodiments, anions with individual negative charge of -2, or -3, or -4 are present in the coordination compound according to the invention, examples a33 to a38 thereof are illustrated in **FIG. 4****.**

Preferably, the at least one lanthanide coordinated by the ligand according to formula (1-1) is Ce3+, Eu2+, or Yb2+. In other words, Ytterbium or Europium in oxidation state +2 preferably are coordinated by an organic ligand represented by the formula (1-1), (1-2), (1-3-1) to (1-3-4). Here, the small, rigid cavity of the ligand according to formula (1-1) provides a size discriminating ligand that stabilizes the divalent over the trivalent oxidation stage of the central cation. In this way, oxidation of the atypical divalent into the common trivalent oxidation state is prevented for the lanthanide. Thus, a metal organic coordination compound is provided that is highly stabilized against oxidation such that processing at ambient conditions becomes possible.

More preferably, the at least one lanthanide coordinated by the ligand according to formula (1-1) is Ce3+, or Eu2+, and even more preference is given to Eu2+ being the lanthanide. Here, preference for Ce3+, Eu2+, or Yb2+ aims to apply the coordination compounds according to the invention as electroluminescent emitters with deep blue light emission. Indeed, all three cations possess sufficiently energetically high intrametallic transitions suitable to generate deep blue electroluminescence. However, in contrast to Ce3+ and Eu2+ with their open shell configuration, Yb2+ has a closed shell configuration with tendency to form meta stable triplet excited states, which are less suitable to generate light with high efficiency. Within the more preferred lanthanides Ce3+ and Eu2+ the latter is even more preferred, because of the more color pure single peak emission spectrum for Eu2+, whilst Ce3+ emits with a less color pure doublet spectrum. Examples of coordination compounds based on Ce3+ and Eu2+ according to the invention are shown in E1 to E50 in **FIG. 5****.**

In various embodiments, the coordination compound according to the invention may be used in a mixture with at least one second electrically neutral or charged organic compound. Preferable for application in electroluminescent devices, the second organic compound preferably has a triplet energy higher than 2.5 eV, more preferably higher than 2.6 eV and even more preferably higher than 2.7 eV to ensure that the excitation is confined at the preferably blue light emitting coordination compound according to the invention. Alternatively, or in addition, the coordination compound may have a higher hole affinity compared to the second organic compound.

In various embodiments, a compound may include the coordination compound according to any of the described or illustrated embodiments and a polymer with a molecular weight Mn above 1000 g/mol. In various embodiments, the coordination compound may be covalently attached to the polymer backbone for example via the side groups **R₁-R₅** in formula (1-1). Alternatively, the covalent link may be formed using a suitable linker in **L₀**, **L₁,** or **L₂.**

In various embodiments, the coordination compound according to various embodiments may be dispersed into a matrix of suitable optical properties, such as for example a high transparency in certain desired parts of the visible spectrum. If this matrix is brought in optical contact to a light source emitting at a sufficiently short wavelength, this light may be absorbed by the coordination compound and reemitted at a substantially longer wavelength. A suitable light source may for example be a light emitting diode emitting light at a wavelength substantially shorter than 430 nm, which may be reemitted by the coordination compound at a wavelength longer than 430 nm. The matrix may have any physical dimensions. It may for example be a thin layer of 10 nm to 10,000 nm. The matrix may as well be of granular form or in form of small particles of average diameter between 10 nm and 100,000 nm. For use in optical devices, in the latter cases, the matrix may be applied inside another host material for support.

The combination of unique emission properties with the ease of processing makes the coordination compounds according to various embodiments highly attractive for application in organic electronic devices. In this description an organic electronic device may be any device or structure including substantially organic layers or subunits, where an electro-magnetic stimulus may be converted into an electrical field or current or vice versa, or where an input electrical current or electrical field may be used to modulate an output electrical current or electrical field.

In one embodiment, the organic electronic device including the coordination compound according to various embodiments may be used as semiconducting organic material in an organic field-effect transistor or an organic thin-film transistor.

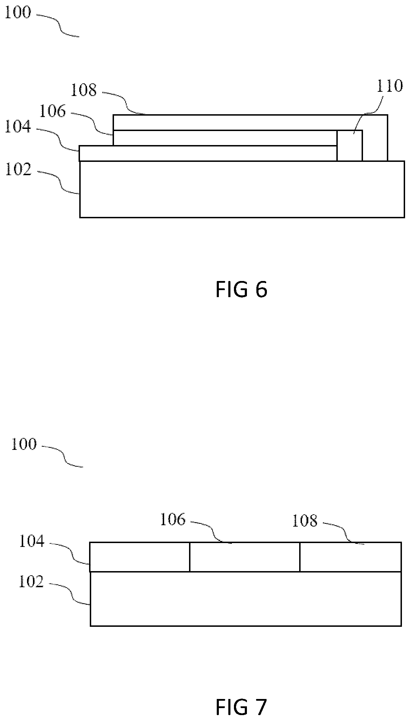

**FIG. 6** and **FIG. 7** illustrate embodiments of an organic electronic device 100 configured as optically active device. The organic electronic device may include a first electrode 104, e.g. on a substrate 102 or as the substrate; a second electrode 108; and an organic layer 106 arranged such that it is electrically interposed between the first and second electrodes 104, 108. The first and second electrodes 104, 108 may be electrically insulated from each other by an insulating structure 110, e.g. a resin or polyimide. The first and second electrodes 104, 108 may be stacked over each other (FIG. 6) or may be arranged in a common plane (FIG. 7).

The organic layer 106 may include the coordination compound according to any of the described or illustrated embodiments, e.g. as a pure compound, in a mixture or in a compound linked to a polymer as described before.

In various embodiments, the organic electronic device 100 may be an optoelectronic device, the optoelectronic device being at least one of an organic light emitting diode (OLED), a light emitting cell (LEC), an organic photodetector, or a photovoltaic cell. That is, photons from an external electromagnetic field may be absorbed in the organic layer 106 and converted into current by means of an electrical field between the first and second electrodes 104, 108. Such a device would be a photodiode (oPD) and its primarily use may be to sense external light. It would be an organic photovoltaic (OPV) device, if the primarily use is to convert light into current.

The organic layer 106 is arranged electrically between the first and second electrodes 104, 108 such that an electronic current may flow from the first electrode 104 through the organic layer 106 to the second electrode 108 and vice versa during operation, e.g. in light emission applications. Alternatively, in photoelectric applications, a charge carrier pair may be formed in the organic layer 106 and charge carriers of the charge carrier pair may be transported to the first and second electrodes 104, 108 respectively. In other words, in light emission applications, upon application of sufficient voltage, holes and electrons are injected from the anode and the cathode, respectively, and drift towards the organic layer 106, where charges of opposite sign recombine to form a short-lived localized excited state. The short-lived excited state may relax to the ground state thereby giving rise to light emission.

The first and second electrodes 104, 108 may be substantially unstructured layers, e.g. for general lighting applications, or may be structured, e.g. for light emitting diodes or transistors for pixels in a display application.

The organic electronic device 100 may be configured to emit substantially monochromatic light such as red, green, blue, or polychromatic light such as white. The light may be emitted through the first electrode 104 (bottom emitter), through the second electrode 108 (top emitter), or through first and second electrodes 104, 108 (bidirectional emitter). The light may as well substantially be emitted in a direction parallel to the organic layer 106 using suitable opaque electrodes 104, 108. In such a layout lasing may be achieved, and the device may be an organic laser, which, in this description, may be considered as a specific type of electroluminescent devices. The coordination compounds according to various embodiments may have excellent emission properties, including a narrow, deep blue emission spectrum with short excited state lifetime. Given its high atomic weight, the optical transitions in particular of Ce3+, and Eu2+ may be as well widely indifferent to excitation with either spin 1 or spin 0 electron-hole pairs. In other words, they will harvest singlet and triplet excitations, giving high light emission efficiency. As such the coordination compounds according to various embodiments may be ideally suited for application in organic electroluminescent devices, such as organic light emitting diodes (OLED). In this description, an electroluminescent device may be any device including an organic layer disposed between and electrically connected to an anode 104/108 and a cathode 108/104. Upon application of sufficient voltage, holes and electrons may be injected from the anode 104/108 and the cathode 108/104, respectively, and drift towards the organic layer 106, where charges of opposite sign recombine to form a short-lived localized excited state. The short-lived excited state may relax to the ground state thereby giving rise to light emission. Relaxation pathways without light emission, such as thermal relaxation, may be possible too, but may be considered undesirable, as they lower the conversion efficiency of current into light of the device. Organic light emitting cells (LECs) may be considered as a subclass of OLED devices comprise of mobile charged species inside the active organic layer 106, which are able to drift inside a external applied electrical field. As such OLEDs encompass LEC type devices.

Further layers may be formed and in electrical connection between the first and second electrodes 104, 108, e.g. configured for charge carrier (electron or hole) injection, configured for charge carrier transport, configured for charge carrier blockage or configured for charge generation. The recombination of electrons and holes may as well happen at the interface of the organic layer 106, which contains the coordination compound according to the invention and an adjacent layer, such an electron blocking layer. Further optically functional layers, e.g. a further electroluminescent material and/or a wavelength conversion material may be formed electrically between the first and second electrodes 104, 108 and in the optical path of the organic layer 106, e.g. on top of the second electrode 108 and/or on the opposite side of the substrate 102. In addition, encapsulation structures may be formed encapsulating the electrically active area, e.g. the area in which an electrical current flows, and may be configured to reduce or avoid an intrusion of oxygen and/or water into the electrically active area. Further optically functional layers, e.g. an antireflection coating, a waveguide structure and/or an optical decoupling layer may be formed within the optical light path of the organic layer 106.

As example, hole or electron blocking layers may be used to optimize the individual hole and electron currents through the organic electronic device 100. This may be known to those skilled in the art as charge balance in order to optimize efficiency and operational stability. In various embodiments, dedicated hole or electron charge transport layers may be present in the organic electronic device 100 to space the emission region from the first and second electrodes 104, 108.

Examples of hole transport materials include known materials such as fluorene and derivatives thereof, aromatic amine and derivatives thereof, carbazole derivatives, dibenzofuran, dibenzothiophene, and polyparaphenylene derivatives. Examples of electron transport materials include oxadiazole derivatives, triazine derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and its derivative, diphenoquinone derivatives, and metal complexes of 8-hydroxyquinoline and its derivatives and various derivatives of silanes.

In various embodiments, those charge transport layers may include electrical dopant molecules or metals, or may be in contact to charge injection layers. Any of those auxiliary layers may be fully organic or may include inorganic functional moieties. For example, charge transport layers may be made of the class of Perovskite materials.

The coordination compound or mixture as illustrated or described in any one of the embodiments may be used as a pure organic emitting layer 106 of any thickness in the range of 0.1 nm and 100 nm, preferably in the range of 0.1 and 10 nm.

The organic layer 106 may in various embodiments include charge transport materials to improve charge transport into and through the organic layer 106. Charge transport materials may be any material that is able to transport either holes or electrons or both types of charges. In particular a charge transport material may be any aryl, or heteroaryl organic compound or any metal complex or any mixture thereof. Examples of materials present in the light emitting layer 106 include carbazole derivatives, including carbazole-phosphine oxide materials, oxadiazole derivatives, triazine derivatives, silane derivatives and any other material or combination of materials with sufficiently high triplet level that may transport charges or otherwise benefit the device performance. The volume percentage of the coordination compound as a function of the combined charge transport materials may be between 0.5 and 99.5 vol% in the organic layer 106.

In various embodiments, the oxidation potential of the coordination compound of the organic layer 106 may be higher compared to all charge transport materials present in the organic layer 106. A wide variety of techniques on how to measure oxidation potentials have been published in the literature. However, for the purpose of various embodiments, the particular technique of how to measure the oxidation potential is not essential, e.g. only the relative order between the coordination compound and the charge transport materials may be of importance. Oxidation potentials may for example be deducted using quantum mechanical computing techniques based on density functional theory and experimental techniques are very well known in the art, e.g. see R.J.Cox, Photographic Sensitivity, Academic Press, 1973, Chapter 15.

The organic layer 106 may contain any other organic or inorganic material in a range of 0.1 to 99.9 vol% that are not intended to transport charges. For example, the organic layer 106 may include polymers (in a mixture or as a compound) which may be added to improve film quality and prevent crystallization. Other materials may be added to evenly space the coordination compound inside the organic layer 106.

In color conversion materials, the coordination compounds according to various embodiments may allow the ease of processing using vacuum based techniques. In other words, the evaporation temperature of the compounds including the lanthanides cation may be sufficiently low to allow for thermal vacuum processing techniques to be used. Reduced evaporation temperatures can be achieved according to the invention by using the metal-organic coordination compounds as here the ionic character of the compounds is strongly suppressed, if compared to similar organic salts without covalently attached monoanionic groups. Consequently, the evaporation temperature is reduced and incorporation into organic electronic devices becomes possible using state-of-the-art vacuum deposition techniques.

The excitation of the light emitting coordination compound may be energetically confined. This way, high efficiencies may be achieved. Thus, confinement layers may be formed adjacent to the organic layer 106, wherein the confinement layers may have a triplet energy T₁ higher than 1.8 eV, e.g. higher than 2.3 eV, e.g. higher than 2.7 eV. Similarly, any material of the organic layer 106 (other than the electroluminescent compound) may have a triplet energy T₁ higher than 1.8 eV, e.g. higher than 2.3 eV, e.g. higher than 2.7 eV.

In various embodiments, the organic electronic device 100 includes two or more subunits each including at least one light emitting layer. The subunits may be stacked over each other physically separated and electrically connected by a charge generation layer or, alternatively, may be arranged side by side. The subunits may be subpixels of a pixel in a display or general lighting application. The light emitted by the subunits may be mixed to generate a light of a predetermined color. Each subunit may emit light of the same or a different color. The overall light emitted by such organic electronic device 100 may contain a narrow spectral region, such as blue, or may contain a wide spectral region such as white, or a combination thereof. The coordination compound illustrated or described in any one of the embodiments may or may not be present in all subunits of the organic electronic device 100.

In various embodiments, the light emitted by the organic electronic device 100 may be in optical contact to at least one optically active layer, including any optically active materials such as organic molecules or quantum dots. The optically active layer may be a spectral filter element, which may absorb part of the light emitted by the organic electronic device 100. In another embodiment, the optically active layer may absorb at least part of the light emitted by the organic electronic device 100 and may reemit it at longer wavelength (wavelength conversion), e.g. by quantum dots.

As example, the organic layer 106 may be configured to emit light substantially at wavelengths shorter than 500 nm and the optically active layer may be configured to substantially reemit light at wavelengths longer than 500 nm. The optically active layer may be placed in between the anode and cathode of the organic electronic device 100 or outside of it. The optically active layer may as well be part of the organic layer 106.

The organic electronic device 100 may be configured as a large area OLED device used for illumination, signage, or as a backlight. Alternatively, the organic electronic device 100 may include a plurality of OLEDs arranged in a pixilated layout (plurality of OLED pixels) that are individually connected electrically, e.g. for flat panel display applications. Here, individual pixels may have the capability of emitting light of substantially narrow spectral portions; especially of red, green, and blue. The coordination compound may or may not be present in any of the individual pixels.

In another embodiment, the individual pixels may be configured to emit white light. Red, green, and blue spectral portions are generated by using suitable filter elements in optical contact with the respective pixelated OLEDs.

In another embodiment, the OLED pixels emit blue light and the red and green spectral portions may be generated by using a suitable color conversion element in optical contact with the OLED pixels.

In another embodiment, the OLED pixels emit substantially in the near-UV spectral region with wavelength <450 nm and the red and green and blue spectral portions may be generated by using a suitable color conversion element in optical contact with the OLED pixels.

In various embodiments, the organic electronic device 100 includes an anode 104; a cathode 108; and an organic layer 106 disposed between the anode 104 and the cathode 108. The organic layer 106 includes an electroluminescent coordination compound according to various embodiments. The coordination compound includes at least one lanthanide coordinated by a cyclic organic ligand according to the present invention according to formula (1-1).

The coordination compound may be imbedded into at least one second electrically neutral organic compound. The second organic compound may have a triplet energy higher than 2.5 eV, more preferably higher than 2.6 eV and even more preferably higher than 2.7 eV. The coordination compound may have a higher hole affinity compared to the second organic compound.

An organic electronic device 100 according to various embodiments may be fabricated using a wide range of commonly used techniques, including, but not limited to, deposition of all or some layer, from gas phase vacuum deposition, solution phase, or gas phase using a carrier gas method.

In various embodiments, deposition via the gas phase in vacuum may be used, whereby the coordination compound may either undergo sublimation or evaporation. The coordination compounds according to the invention show an improved sublimation/evaporation yield. The transfer into the gas phase may be improved by using a carrier gas technology, whereby an inert gas that may not be deposited into the organic layer may be helping the sublimation or evaporation of the coordination compound to be deposited. During the deposition process from gas phase, the coordination compound may as well be co-deposited with one or more material to fabricate any desired mixed layers.

Another preferred technique to fabricate layers including the coordination compound according to various embodiments may be deposition from a liquid phase using a mixture or a single organic solvent, whereby the coordination compound according to various embodiments may be dissolved or forms a suspension within the organic solvent; in this description may be referred to as the ink. The ink using this deposition process, may include a wide variety of other materials apart from the coordination compound according to various embodiments to allow fabrication of mixed layers from solution. Additives within the ink may for example, but may not be limited to, be organic or inorganic materials capable of transporting charges, materials that improve the film formation, materials that improve the distribution of the coordination compound within a host material, organic or inorganic materials that improve the efficiency of the device, e.g. by reducing the refractive index. The deposition from solution may not be limited to any specific technique. Examples of the deposition from solution include spin coating, casting, dip coating, gravure coating, bar coating, roll coating, spray coating, screen printing, flexographic printing, offset printing, inkjet printing. The improved solubility of the coordination compounds according to the present invention in non-polar solvents eases the deposition from solution and makes it advantageous.

Various post processing techniques may be applied to improve the performance or stability of the organic electronic device. In one embodiment, some or all layers of the organic electronic device include functional groups capable of chemically crosslinking upon thermal or optical exposure thereby forming larger covalently bound molecules with improved physical properties.

In various embodiments, the coordination compound may be formed in-situ using deposition from solution. Here, the organic ligand according to formula (1-1) of the present invention including but excluding the lanthanide metal, may be first deposited onto a suitable substrate or other organic layer thereby forming a seed layer. Any suitable technique may be used to fabricate this seed layer. This seed layer including the organic ligand may include any other material. Preferred may be organic charge transport materials, for example suitable to achieve hole transport for organic electronic devices. Preferred may be further additional inert organic or inorganic materials that aid the layer formation or improve its thermal stability or improve the distribution of the organic ligand within this seed layer. In a next deposition step and sequential to forming the seed layer including the organic ligand according to the invention excluding the metal, a layer including a molecular salt including comprising the lanthanide may be fabricated using a solution process. The lanthanide compound may be any charge neutral compound including a lanthanide in an oxidation state 2+, 3+, 4+ and any number of suitable anions. The anions may as well be covalently bound to a suitable polymer. The ink including the lanthanide compound may as well include one or more additive to fabricate mixed layers. These additives may be organic or inorganic materials to aid charge transport, may be organic or inorganic materials that improve the efficiency of the device, or may be any material that improves the film formation. At the interface of the seed layer including the organic ligand according to the invention, the solubilized lanthanide metal compound will interact with the organic ligand according to formula (1-1) to form the coordination compound according to various embodiments in-situ. Alternatively, the process of first forming the layer containing the ligand and secondly processing the lanthanide compound may be inverted.

Preferably, the metal-organic coordination compound is used directly.

For one or more aspects, at least one of the components set forth in one or more of the preceding figures may be configured to perform one or more operations, techniques, processes, and/or methods as set forth in the example section below.

### EXAMPLES

The examples set forth herein are illustrative and not exhaustive.

Any of the examples may be combined with any other example (or combination of examples), unless explicitly stated otherwise. The foregoing description of one or more implementations provides illustration and description, but is not intended to be exhaustive or to limit the scope of aspects to the precise form disclosed. Modifications and variations are possible in light of the above teachings or may be acquired from practice of various aspects. Any of the above-described examples may be combined with any other example (or combination of examples), unless explicitly stated otherwise.

Each of the substituents shown in Figures 2 and 3 can be combined with any of the polycyclic organic ligands depicted in Figures 1 and 5. The polycyclic organic ligand shown in formulae (1-1), (1-2), (1-3-1) to (1-3-4) and (1-4) can have a basic ring structure as depicted in Figures 1 and 5. Consequently, each element or substituent depicted in any of Figures 1 to 3 and 5 can be combined or used for restricting the stated general formulae. In the following route1, the meaning of -Bn for R has the meaning benzyl, as depicted in Figure 1, L 11.36. Especially, organyl groups R₁ can be introduced by N-methylation, N-alkylation, reductive N-alkylation or N-alkylation with oxiranes.

While the invention has been particularly shown and described with reference to specific aspects, it should be understood by those skilled in the art that various changes in form and detail may be made therein without departing from the spirit and scope of the invention as defined by the appended claims. The scope of the invention is thus indicated by the appended claims and all changes which come within the meaning and range of equivalency of the claims are therefore intended to be embraced.

### Synthesis examples according to the present invention

The following procedures are, unless indicated otherwise, carried out under an inert atmosphere in oven dried glassware. The solvents, starting materials and reagents can be purchased from any commercial source and are, unless otherwise stated, used without further purification.

### Synthesis of the starting materials.

Europium borohydride-tetrahydrofuran complex

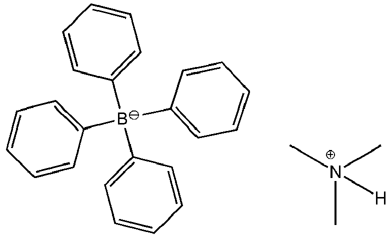

The solution of trimethylamine hydrochloride (1.1 eq, 1.08 g, 11.3 mmol) in 10 ml of deionized water was dropwise added to the solution of sodium tetraphenylborate (1 eq, 3.52 g, 10.3 mmol) in 50 ml of deionized water. The white precipitate was separated by suction filtration, washed with deionized water and dried in vacuum at 75 °C overnight.

Yield: 3.2 g (81.9 %)

### Trimethylammonium tetrafluoroborate

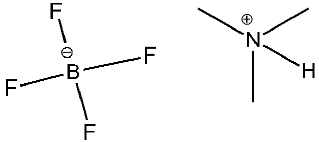

Tetrafluoroboronic acid (1 eq, 70.1 g, 399 mmol) was neutralized with an excess of aqueous solution of trimethylamine (2 eq, 94.3 g, 798 mmol) at 0 °C. The obtained solution was evaporated to dryness, the residue was dissolved in hot methanol (120 ml), filtered and allowed to cool down to RT overnight. The precipitated product was separated by filtration, washed with cold methanol and dried in vacuum at 50 °C overnight.

Yield: 85.3 % (colorless crystals)

### Trimethylammonium dodecamethylcarbadodecaborate

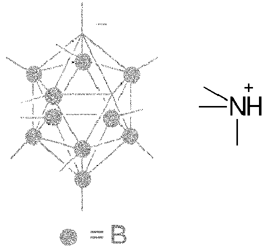

The title compound was prepared using a known procedure (Inorganic Syntheses, Volume 35, edited by Thomas B. Rauchfuss, p.57-59). The product was dried in vacuum at 100 °C before use.

### Bis[bis (trimethylsilyl)amide]bis (tetrahydrofuran)europium(II) (Eu(HMDS)₂)

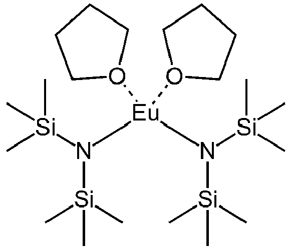

Sodium bis(trimethylsilyl)amide (2 eq, 9.5 g, 49.2 mmol) was added under a nitrogen atmosphere to the solution of europium diiodide (1 eq, 10 g, 24.6 mmol) in 300 ml of anhydrous THF. The mixture was stirred at RT overnight. THF was removed in vacuum and residue was extracted with hexane (250 ml). The extract was filtered and concentrated to ca. 50 ml volume. Cooling of the solution to 0 °C resulted in precipitation of the title product as orange-yellow crystals.

Yield: 90% (13.77g)

### Europium (II) carbadodecaborate (Eu(CB)₂)

Trimethylammonium carbadodecaborate (Katchem, 1 eq, 813 mg, 4 mmol) was dried in a Glovebox for 1 h at 100 °C, then dissolved in anhydrous THF (20 ml). A solution of bis[bis(trimethylsilyl)amido]bis(tetrahydrofuran)europium (0.5 eq, 1234 mg, 2 mmol) in anhydrous THF (10 ml) was added dropwise at RT. The mixture was stirred for 1 h. White crystals formed which were collected via suction filtration, washed with THF and dried in vacuum to yield the title compound.

### Europium (II) dodecamethylcarbadodecaborate Eu(MCB)₂

A solution of bis[bis(trimethylsilyl)amido]bis(tetrahydrofuran)europium (0.5 eq, 1.23g, 2 mmol) in 15 ml of diethyl ether was added to the solution of trimethylammonium dodecamethylcarbadodecaborate (1 eq, 1.51 g, 4.06 mmol) in 20 ml of anhydrous diethyl ether. The mixture was stirred for 1 h at RT. The precipitate was collected by suction filtration, washed with diethyl ether and hexane and dried in vacuum at RT. The title product was obtained in nearly quantitative yield.

### Trimethylammonium tetraphenylborate Eu(BH4)₂×(THF)ₙ

A solution of sodium methoxide (266mg, 4.93mmol, 2.2eq) in 30ml of anhydrous methanol was added to a solution of europium(II)chloride (499mg, 2.24mmol, 1eq) in 70ml of anhydrous methanol under nitrogen atmosphere. Reaction mixture was stirred at RT for 1h, then solvent was evaporated in vacuum. Residue was dried at 100°C in vacuum for 1h and re-dispersed in anhydrous THF (100ml). Borane-tetrahydrofurane complex solution (1M, 8.96ml, 8.96mmol, 4.4eq.) was added dropwise and the mixture was stirred at RT overnight. Turbid solution was filtered through sintered glass filter (pore 5), solid on filter washed with 3 portion of anhydrous THF (20ml). Combined filtrate was evaporated to dryness giving the title product as pale yellow powder in nearly quantitative yield.

### Europium(II) pyrazolate (Eu(Prz)2)

To a solution of pyrazole (68mg, 1.00mmol, 2.0eq) in 2.5ml anhydrous THF was added a solution of bis[bis(trimethylsilyl)amido]bis(tetrahydrofuran)europium (308mg, 0.50mmol, 1.0eq) in 2.5ml THF. The mixture was stirred at rt for 2h. The solution was concentrated and the residue diluted with 5ml of a 1:1-mixture of n-hexane and Et₂O. The precipitate was filtered, washed with Et₂O and dried in vacuum. The title product was obtained in nearly quantitative yield. rt = room temperature

### Bis(3,5-Dimethyl-1H-pyrazol-1-die)europium(II) (Eu(3,5-DiMePru)₂

A solution of Bis[bis ( trimethylsilyl)amide]bis (tetrahydrofuran)europium(II) (Eu(HMDS)₂) (1000 mg, 1.62 mmol, 1 eq) in 16 ml of anhydrous THF were added to a solution of 3,5-dimethylpyrazole in 36 ml of anhydrous THF. The mixture was stirred at 50 °C for 2 h. The solvent was removed under reduced pressure. The residue was diluted with diethyl ether and stirred for 1 h at RT. The precipitated solid was filtered through a sintered glass filter (pore 4). The resulting solid has been washed with diethyl ether as well as hexane and dried in vacuum at RT. The title product was obtained in nearly quantitative yield as a yellow powder.

Yield: 79 % (438 mg). RT = room temperature

### Europium(II) bis(oxo(trifluoromesylimino)(trifluoromethyl)-λ6-sulfanolate (Eu(TFSI)₂)

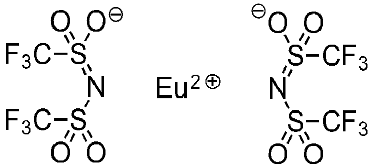

Europium(II) chloride (1 eq, 2.01 g, 9 mmol) was dissolved in MeOH (18 ml). A solution of sodium methoxide (2 eq, 0.972 g, 18 mmol in MeOH 18 ml was added and the mixture was stirred for 30 min at rt. A solution of trifluoromethansulfonimide (2 eq, 5.33 g, 18 mmol) in MeOH (18 ml) was added and stirring continued for 30 min. The solvent was removed and the residue dried in vacuum. The white precipitate was diluted with THF (ca. 60 ml), filtered and washed with THF (3x 20 ml). The filtrate was concentrated and dried in vacuum. The residue was diluted with Et₂O (ca. 40 ml) and the mixture was stirred at rt for 30 min. The precipitate was filtered, washed with Et₂O and dried in vacuum. Yield: 88% (5.65g).

### Bis((bis(perfluoropropyl)phosphoryl)oxy)europium(Eu(PFP)₂)

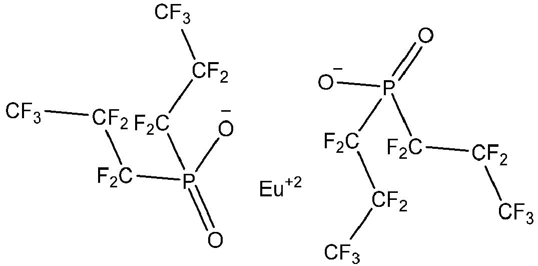

Bis(perfluoropropyl)phosphinic acid was synthesized by a known procedure (Dalton Trans., 2012, 41, 13504).

Europium(II) chloride (1 eq, 0,208g, 0,934 mmol) was dissolved in MeOH (10 ml). A solution of sodium methoxide (2 eq, 0.101 g, 1.87 mmol) in MeOH (5 ml) was added and the mixture was stirred for 30 min at rt. The solution was evaporated to dryness and additionally dried at 70°C in vacuum. The residue (pale orange solid) was suspended in THF (20ml) and bis(perfluoropropyl)phosphinic acid (2 eq. 0.751g, 1,87mmol) was added dropwise. The mixture is stirred for 1h at room temperature, then filtered through a syringe filter. The filtrate, containing the title compound, is used for the synthesis of the emitter.

Ligands, used for the synthesis of Eu(II) complexes, were synthesized according to the following general scheme:

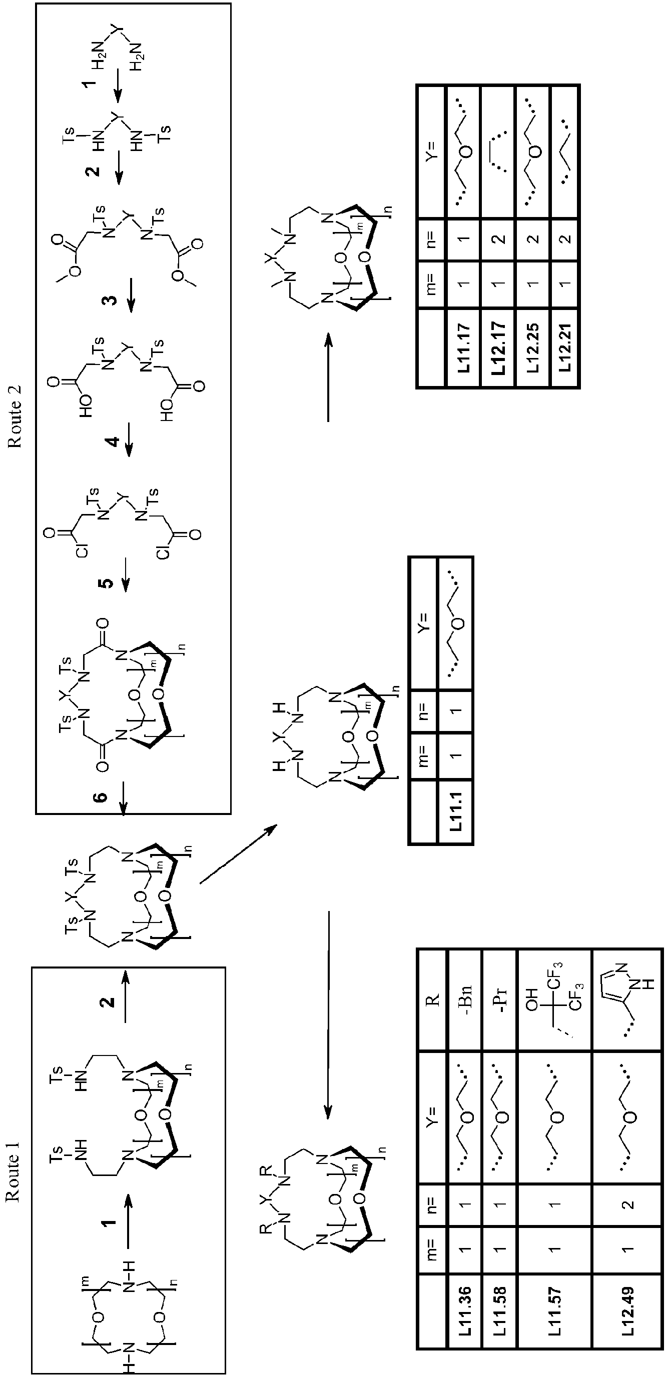

General procedures.

The synthesis of the aza-crown ethers can follow per se known procedures as they are for example described in J. CHEM. SOC. PERKEN TRANS. 1, 1988, pages 3141 to 3147. The general procedures followed in the synthesis of the coordination compounds of the present invention are described in the following:

### Route1. Step1. Synthesis of bis[2-(p-tolylsulphonylamino)ethyl]-oxa-diazacycloalkanes

A solution of Oxa-diazacycloalkane (1 eq, 22.9 mmol) in MeCN (110 ml) was heated to reflux. N-Tosylaziridine (2 eq, 45.8 mmol), dissolved in MeCN (120 ml), was added dropwise within 2.5 h. The mixture was cooled to RT and stirred further for 16 h. The solvent was removed in vacuum. The residue was purified by recrystallization from an appropriate solvent (ethanol, ethyl acetate or toluene) or by filtration through a silica plug (iso-propanol) followed by recrystallization from the appropriate solvent. Following compounds were prepared using this method:
1-(Tosylamino)-2-(10-[2-(tosylamino)ethyl]-1,7-dioxa-4,10-diaza-4-cyclododecyl)ethane : 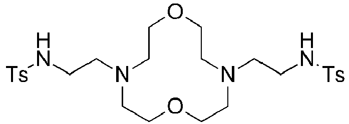 Yield: 95%; 1H-NMR (60 MHz, CDCl3): δ (ppm) = 2.25-3.3 (br.m. 22 H), 3.64 (br. t., 8 H), 6.79 (br. s., 2 H), 7.27 (d, J=8.09 Hz, 4 H), 7.75 (d, J=8.39 Hz, 4 H); MS (ESI, ACN): m/z =569.4 [M+H]+, 592.4 [M+Na]+
1-(Tosylamino)-2-(13-[2-(tosylamino)ethyl]-1,4,10-trioxa-7,13-diaza-7-cyclopentadecyl)ethane : 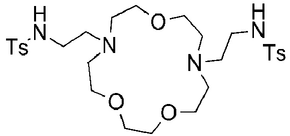 Yield: 95%; 1H-NMR (60 MHz, CDCl3): δ (ppm) = 2.42 (s, 6 H), 2.47-2.80 (m, 12 H), 2.80-3.12 (m, 4 H), 3.35-3.74 (m, 12 H), 6.19 (s, 2 H), 7.12-7.93 (dd, 8 H); MS (ESI): m/z = 613.4 [M+H]+, 635.4 [M+Na]+

### Route1. Step2. Synthesis of bis(p-tolylsulphonyl)-diazacryptandes.

A mixture of bis[2-(p-tolylsulphonylamino)ethyl]-oxa-diazacycloalkane (1 eq, 10 mmol), dibromide or ditosylate (1 eq, 10mmol), tetrabutylammonium iodide (0.28 eq., 2.8 mmol), NaOH (266 eq, 2.66 mol, as 50 wt-% aqueous solution) and toluene (600 ml) was stirred under reflux for 12-16 h. The mixture was cooled to rt and transferred to separation funnel. The organic layer is separated and dried with MgSO₄. The solvent was removed in vacuum and the residues purified by flash-chromatography using CH₂Cl₂/MeOH or by recrystallization from an appropriate solvent (acetonitrile, toluene or ethyl acetate)

Following compounds were prepared using this method:
4,10-Ditosyl-7,16,21-trioxa-1,4,10,13-tetraazabicyclo[11.5.5]tricosane: 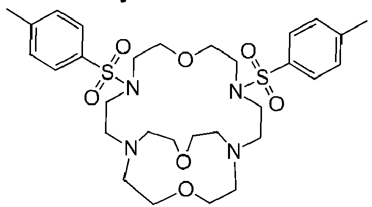 Yield: 92%; 1H-NMR (60 MHz, CDCl3): δ (ppm) = 2.2-3.0 (m, 18 H), 3.1-3.9 (m, 14 H), 7.3 (d, 4 H), 7.6 (d, 4 H), MS (ESI -ACN): m/z = 639.5 [M+H]+
4,10-Ditosyl-7,16,19,24-tetraoxa-1,4,10,13-tetraazabicyclo[11.8.5]hexacosane: 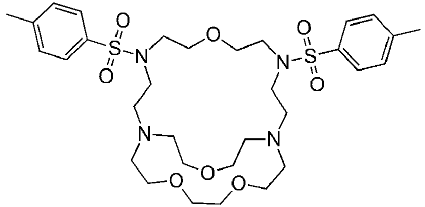 Yield: 40 %; 1H-NMR (60 MHz, CDCl3): δ (ppm) = 2.53 (s, 6 H), 2.61-2.99 (m, 12 H), 3.18-3.81 (m, 24 H), 7.26-7.98 (dd, 8 H); ; 13C-NMR (CDCl3, 15 MHz): δ (ppm) = 21.5 (CH3), 48.4 (CH2), 55.2 (CH2), 55.9 (CH2), 69.6 (CH2), 70.9 (CH2), 75.0 (CH2), 77.1 (CH2), 79.2 (CH2), 127.4 (CH), 129.9 (CH), 143.2 (Cq); 2 MS (ESI): m/z = 683.3 [M+H]+, 705.4 [M+Na]+.
4,8-Ditosyl-14,17,22-trioxa-1,4,8,11-tetraazabicyclo[9.8.5]tetracosane : 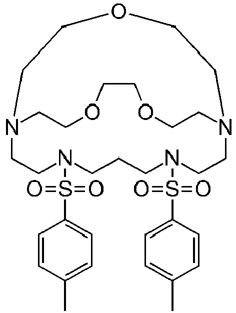 Yield: 40 %, 1H-NMR (60 MHz, CDCl3): δ (ppm) = (m, 2 H), 2.50 (s, 6 H), 2.54-2.94 (m, 12 H), 3.07-3.43 (m, 6 H), 3.43-3.78 (m, 12 H), 7.19-7,7.92 (dd, 8 H). 3.

### Route2. Step1. Preparation of Tosylamides

The diamine (1 eq, 60 mmol) is placed in a round-bottom flask. The substance is dissolved in 300 ml dry pyridine and cooled to 0 °C. 4-Methylbenzenesulfonyl chloride (2.05 eq, 123 mmol) is added under nitrogen counter flow and the temperature is raised to room temperature overnight. The reaction mixture is poured into 1000 ml of water. The resulting solid is filtered, washed with water and dried. The obtained crude product is further purified by recrystallization from an appropriate solvent.

Following compounds were prepared using this method:
1,2-Bis(tosylamino)ethane

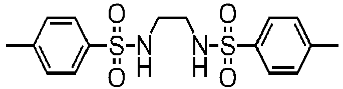

Yield 75%; 1H-NMR (60 MHz, CDCl3): δ (ppm) = 2.16 (s, 6 H), 2.52 (s, 4 H), 3.14 (s, 2 H), 7.07-7.49 (m, 8 H); 13C-NMR (DMSO-d6, 15.53 MHz): δ (ppm) = 21.20 (CH3), 42.41 (CH2), 126.72 (CH), 129.90 (CH), 137.59 (Cq), 142.99 (Cq); MS (ESI): m/z = 391.0 [M+Na]+, 759.0 [2M+Na]+

### Route2. Step2. Tosylamide extention with methylbromoacetate

151 ml methanol is placed in a two-neck round-bottom flask, equipped with a reflux condenser. Sodium methoxide (80.9 mmol, 2.1 eq) is added at 0 °C under nitrogen counter-flow. The mixture is stirred at 0 °C until the sodium methoxide is fully dissolved. The bis(tosylamide) (38.5 mmol, 1 eq) is added at this temperature and stirred under reflux conditions for 2h. The methanol is removed at reduced pressure. In order to remove methanol traces, the residue is suspended in an anhydrous toluene and the solvent is removed by vacuum distillation. This step is repeated twice. The white, solid disodium salt of bis(tosylamide) is finally dried under reduced pressure and elevated temperature.

Methyl bromoacetate (308 mmol, 8 eq) is placed in two-neck round-bottom flask equipped with a reflux condenser at 0 °C. The disodium salt of bis(tosylamide) is added under nitrogen counter flow and the mixture is stirred at room temperature for 60 min. After this time, the reaction mixture is heated up to 50 °C and stirring is continued for further 60 min. Volatiles are removed in vacuum (p= 16 mbar, T= 60 °C) and the residue is dissolved in 200 ml chloroform and 80 ml water. The organic layer is separated, washed three times with water and one time with brine, dried over magnesium sulfate and evaporated to dryness. The residue is recrystallized form methanol, separated by filtration and washed with ice-cold methanol, yielding the product as white powder.

Following compounds were prepared using this method:
Methyl ({2-[(methoxycarbonylmethyl)-*N*-tosylamino]ethyl}-*N*-tosylamino)acetate

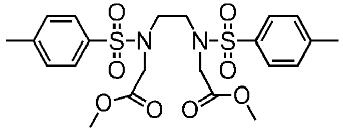

Yield 92%; 1H-NMR (DMSO-d6, 61.75 MHz), δ (ppm) = 2.21 (s, 6 H), 3.08 (s, 4 H), 3.38 (s, 6 H), 3.90 (s, 4 H), 7.14-7.55 (m, 8 H); 13C-NMR (DMSO-d6, 15.53 MHz): δ (ppm) = 21.39 (CH3), 47.37 (CH2), 49.65 (CH3), 52.27 (CH2), 127.41 (CH), 130.16 (CH), 136.47 (Cq), 143.92 (Cq), 169.93 (Cq); MS (ESI): m/z = 535.0 [M+Na]+.

### Route2. Step3. Hydrolysis of bis(methyl ether)

Bis(methyl ester) (1 eq, 11.9 mmol) is placed in 100 ml three-neck round-bottom flask equipped with reflux condenser. 29 ml of acetic acid (42 eq, 502 mmol) and 7.2 ml conc. hydrochloric acid (6.7 eq, 80.1 mmol) are added and the mixture is stirred for 21 h under reflux. The reaction mixture is cooled to room temperature, precipitated product is filtered off, washed with cold acetic acid and dried yielding the target bis(carboxylic acid) as a white powder. Following compounds were prepared using this method:

### 2,2'-(Ethane-1,2-diylbis(tosylazanediyl))diacetic acid

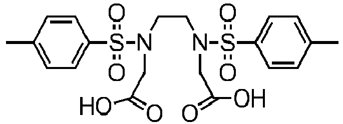

Yield: 85%, 1H-NMR (DMSO-d6, 61.75 MHz), δ (ppm) = 2.16 (s , 6 H), 3.05 (s,4 H), 3.76 (s, 4 H), 7.08-7.51 (m, 8 H), 12.91 (s.br, 2H); 13C-NMR (DMSO-d6, 15.53 MHz): δ (ppm) = 21.21 (CH3), 47.32 (CH2), 49.57 (CH2), 127.19 (CH), 129.94 (CH), 136.56 (Cq), 143.55 (Cq), 170.67 (Cq); MS (ESI): m/z = 507.10 [M+Na]+, 483.05 [M-H]-, 967.29 [2M-H]-

### Route2. Step4. Synthesis of bis(carboxylic acid chloride)

A 100 ml three-neck round-bottom-flask equipped with thermometer and reflux condenser with attached gas outlet, is charged with the dicarboxylic acid (6,7 mmol, 1 eq) and thionyl chloride (33.5 ml, 462 mmol, 68.8 eq). The mixture is stirred under reflux conditions for 6 h until the diacid is fully dissolved whereby the internal temperature shall not exceed 80 °C. Excess of thionyl chloride is removed completely under reduced pressure whereby the temperature shall not exceed 60 °C. The white residue is recrystallized with benzene. After crystallization the residue is filtered off and washed with cold benzene yielding the product as a white powder.

Following compounds were prepared using this method:
2,2'-(ethane-1,2-diylbis(tosylazanediyl))diacetyl chloride

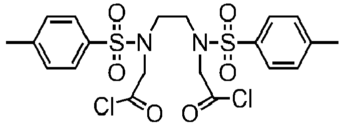

Yield: 78%, 1H-NMR (DMSO-d6, 61.75 MHz), δ (ppm) = 2.27 (s, 6 H), 3.18 (s, 4 H), 3.88 (s, 4 H), 7.18- 7.65 (m, 8 H); 13C-NMR (DMSO-d6, 15.53 MHz): δ (ppm) = 21.34 (CH3), 47.49 (CH2), 49.71 (CH2), 127.33 (CH), 130.05 (CH), 136.70 (Cq), 143.69 (Cq), 170.62 (Cq);MS (ESI): m/z = 521.1 [M+H]+.

### Route2. Step5. Synthesis of diamido [2.2.1] cryptand.

1L three-neck flask, equipped with a large stirring bar and two dropping funnels is charged with 196 mL of CH2Cl2/toluene (4/1 vol.) mixture. One of the dropping funnel is charged with a solution of 1,4,10-trioxa-7,13-diazacyclopentadecane (1 eq, 3.91 mmol) and anhydrous triethylamine (5 eq, 2.72 ml, 19.56 mmol), in 196 ml of CH2Cl2/toluene (4/1 vol.) mixture. The second funnel is charged with a solution of bis(acetyl chloride) (1 eq, 3.91 mmol) in 196 ml CH2Cl2/toluene (4/1 vol.) mixture. Both solutions are added simultaneously at 0 °C to the reaction vessel over a period of 4 h and 15 min with the same addition rate. The reaction mixture is allowed to warm up to room temperature overnight. After 21 h the solvents are evaporated under reduced pressure and the residue is dissolved in 200 ml CHCl3. The solution is washed with water (3 × 100 ml), dried over magnesium sulfate and evaporated to

dryness. The resulting white-yellowish residue is extracted several times with hot toluene. The extracts are combined and evaporated to dryness yielding a title product as colorless, glassy solid.

Following compounds were prepared using this method:
13,16-ditosyl-4,7,21-trioxa-1,10,13,16-tetraazabicyclo[8.8.5]tricosane-11,18-dione

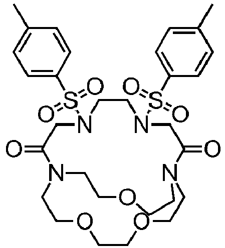

Yield: 51%, 1H-NMR (CDCl3, 61.75 MHz), δ (ppm) = 2.46 (s, 6 H), 2.46-4.12 (m, br.), 4.30-4.79(m, br. 4 H), 7.32 (d., 4 H), 7.75 (d, 4 H), 13C-NMR (CDCl3, 15.53 MHz): δ (ppm) = 21.55 (CH3), 46.69-51.89 (CH2), 67.52-70.20 (CH2), 125.33-129.82 (CH), 135.61-136.61 (Cq), 142.95-143.51 (Cq), 168.06-169.18 (Cq); MS (ESI): m/z = 688.99 [M+Na]+.

### Route2. Step6. Synthesis of diaza-[2.2.1] cryptand.

Diamide [2.2.1]-cryptand (2 mmol, 1 eq) is placed in 100 ml round-bottom flask and dissolved in THF (22 ml) at 0 °C. This solution is treated dropwise with 24 ml BH3*THF solution (23,9 mmol, 12 eq, 1 M in THF). After addition completed, the reaction mixture is heated up to 65 °C . After complete conversion (up to 12 h) of the starting material, the mixture is cooled to 0 °C. Water is added until the gas evolution ceased. The solvents are removed under reduced pressure and the residue is dissolved in 10 ml 6 N HCl and heated to 90 °C for 3 h. This reaction mixture is treated with sat. LiOH-solution until pH 9-10. The mixture is extracted with chloroform (5 × 15ml), combined organic phases are dried over magnesium sulfate, filtered, and evaporated to dryness. The residue is extracted with hot toluene, the extract is filtered and evaporated to dryness yielding the title product as a white powder.

Following compounds were prepared using this method:
13,16-Ditosyl-4,7,21-trioxa-1,10,13,16-tetraazabicyclo[8.8.5]tricosane

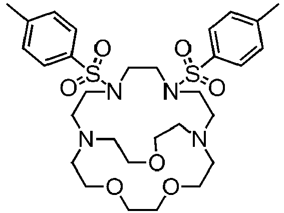

Yield: 82%, 1H-NMR (CDCl3, 61.75 MHz), δ (ppm) = 2.55-2.96 (broad m, 18 H), 3.20-3.80 (broad m, 20 H), 7.41 (d, 4 H), 7.87 (d, 4 H), 13C-NMR (CDCl3, 15.53 MHz): δ (ppm) = 21.62 (CH3), 45.06 (CH2), 49.30 (CH2), 54.77 (CH2), 56.54 (CH2), 57.23 (CH2), 69.47 (CH2), 70.15 (CH2), 70.91 (CH2), 127.59 (CH), 129.74 (CH), 136.37 (Cq), 143.24-143.67 (Cq), MS (ESI): m/z = 639.4 [M+H]+, 645.4 [M+Li]+, 661.3 [M+Na]+.

Cleavage of the tosyl-groups.

A solution of Bis(p-tolylsulphonyl)-cryptand (1 eq, 3.95 mmol) in THF (40 ml) was cooled to T <-78 °C while being stirred. Sodium naphthalene in THF (5 eq, 39.5 ml, 19.8 mmol, 0.5 mol/l) was added dropwise via syringe. After the addition was completed, the mixture was stirred for 2-3 h while being allowed to slowly warm to - 30 °C. The reaction was quenched by adding 1 M HClaq. (ca. 30 ml) until pH 1-2 was reached. The layers were separated. The aqueous layer was extracted with EtOAc (3x 40 ml). The combined extracts were washed with water (30 ml) and brine (30 ml). The combined aqueous layers were alkalized with NaOHaq. (50 wt-%, 12 ml) to pH 13 and extracted with CHCl3 (6x 40 ml). The combined organic layers were dried over MgSO4 and concentrated in vacuum. The crude product was further purified by bulb-to-bulb distillation.

Following compounds were prepared using this method:
7,16,21-Trioxa-1,4,10,13-tetraazabicyclo[11,5.5]tricosane (FIG 1, L11.1).
   Yield: 40.2%, 1H NMR (62 MHz, CDCl3) □ (ppm) = 2.38-3.21 (m,22H) 3.42-4.04 (m, 12H);MS (ESI): m/z = 331.4 [M+H]+, 353.4 [M+Na]+.
4,7,21-Trioxa-1,10,13,16-tetraazabicyclo[8.8.5]tricosane(FIG 1, L12.1)
   Yield: 100 %, 1H-NMR (CDCl3, 61.75 MHz), δ (ppm) = 2.35-3.06 (br. m, 20 H), 3.40-4.04 (br. m, 12 H); 13C-NMR (CDCl3, 15.53 MHz): δ (ppm) = 46.34-48.72 (CH2), 54.45-56.17 (CH2), 67.21-70.81 (CH2), MS (ESI): m/z = 331.3 [M+H]+, 353.2 [M+Na]+.
7,16,19,24-Tetraoxa-1,4,10,13-tetraazabicyclo[11.8.5]hexacosane (FIG 1, L12.9)
   Yield: 95 %, 1H-NMR (60 MHz, CDCl3): δ (ppm) = 2.44-3.05 (m, 28 H), 3.55-3.90 (m, 10 H); MS (ESI): m/z = 375.2 [M+H]+, 397.2 [M+Na]+.
14,17,22-Trioxa-1,4,8,11-tetraazabicyclo[9.8.5]tetracosane (FIG 1, L12.5)
   Yield: 96 %, 1H-NMR (60 MHz, CDCl3): δ (ppm) = 2.44-3.00 (m, 22 H), 3.50-3.90 (m, 12 H); MS (ESI): m/z = 345.2 [M+H]+, 367.2 [M+Na]+.

General procedure for Step 4: N-Methylation.

Oxa-tetraazabicycloalkane (1 eq, 2.3 mmol) was stirred with formaldehyde (42.9 eq, 8014 mg, 98.8 mmol, 37 %) and formic acid (57.6 eq, 6100 mg, 133 mmol) under reflux overnight. The mixture was concentrated in vacuum and the residue alkalized with NaOHaq. (12.5 M) until pH 13 was reached. The aqueous layer was extracted several times with CHCl₃. The combined extracts were dried over Na₂SO₄ and concentrated in vacuum. The residue was extracted with hexane (5x) and the hexane fraction evaporated to dryness, yielding the methylated product as colorless to pale-yellow oil. Final purification was achieved by distillation using a Kugelrohr-apparatus.

4,10-Dimethyl-7,16,21-trioxa-1,4,10,13-tetraazabicyclo[11.5.5]tricosane (FIG 1, L11.17):
Yield: 63.9 %, 1H-NMR (60 MHz, CDCl₃): δ (ppm) = 2.31-2.55 (s, 6 H), 2.61-2.90 (m, 10 H), 3.49-3.82 (m, 16 H); MS (ESI): m/z = 359.3[M+H]+, 381.3 [M+Na]+.

### 13,16-dimethyl-4,7,21-trioxa-1,10,13,16-tetraazabicyclo[8.8.5]tricosane (FIG 1, L12.17)

Yield: 63.9 %, 1H-NMR (60 MHz, CDCl3): δ (ppm) = 2.18-2.29 (m, 6 H), 2.43-2.64 (m, 20 H), 3.33-3.61 (m, 12 H), 13C-NMR (CDCl3, 15.53 MHz): δ (ppm) = 54.89-56.78 (CH2), 67.13-69.86 (CH2), MS (ESI): m/z = 359.2 [M+H]+, 381.2 [M+Na]+.

### (1R,13S)-4,10-Dimethyl-7,16,19,24-tetraoxa-1,4,10,13-tetraazabicyclo[11.8.5]hexacosane (FIG 1, L12.25):

Yield 56 %; 1H-NMR (60 MHz, CDCl3): δ (ppm) = 2.41 (s, 6 H), 2.61-2.90 (m, 20 H), 3.49-3.82 (m, 16 H),; MS (ESI): m/z = 403.2 [M+H]+, 425.2 [M+Na]+.

### (1R,11S)-4,8-Dimethyl-14,17,22-trioxa-1,4,8,11-tetraazabicyclo[9.8.5]tetracosane (FIG 1, L12.21):

Yield: 70 %; 1H-NMR (60 MHz, CDCl3): δ (ppm) = 1.68-2.00 (m, 2 H), 2.36 (s, 6 H), 2.61-2.90 (m, 20 H), 3.54-3.83 (m, 12 H),; MS (ESI): m/z = 379.2 [M+Li]+.

### N-Alkylation with chloromethylpyrazole

To a solution of Oxa-tetraazabicycloalkane (1 eq, 0.236 mmol) in MeCN (12.5 ml) was added N-Ethyldiisopropylamin (4.5 eq, 145 mg, 1.13 mmol). The solution was stirred for 5 min at rt. 3-(Chloromethyl)pyrazole hydrochloride (2 eq, 72 mg, 0.471 mmol) was added in one portion and the mixture stirred for 90 min at 70 °C. The mixture was cooled to rt and 1,8-Diazabicyclo[5.4.0]undec-7-ene (2.5 eq, 95.1 mg, 0.625 mmol) was added. Stirring was continued for 1 d at 70 °C. The solvent was removed in vacuum, the residue diluted with CHCl3 (10 ml) and washed with water (5 ml). The organic layer was concentrated in vacuum and the residue purified by flash-chromatography using a gradient of CH2Cl2/i-propanol (1% to 100%).

Following compounds were prepared using this method:
4,1 0-Bis[(3-pyrazolyl)methyl]-7, 16, 19,24-tetraoxa-1,4, 10,13-tetraazabicyclo[11.8.5]hexacosane (FIG 1, L12.49):
Yield: 37 %; 1H-NMR (60 MHz, CDCl₃): δ (ppm) = 11.16 (s, 2 H), 7.55 (d, 2 H), 6.03 (d, 2 H), 3.75 (s, 4 H), 3.49-3.68 (m, 16 H), 2.38-2.76. (m, 20 H); MS (ESI): m/z = 535.7 [M+H]+, 557.4 [M+Na]+.

### Reductive N-alkylation.

Oxa-tetraazabicycloalkane (1 eq, 0.5 mmol) and the corresponding aldehyde (2 eq. 1.0 mmol) were mixed in 1,2-dichloroethane (20 ml) and treated with sodium triacetoxyborohydride (1.1 mmol). The mixture was stirred at RT under a N2 atmosphere for 12h. The reaction mixture was quenched by adding saturated, aqueous solution of NaHCO3. The product was extracted with chloroform (5 × 10ml). The combined extracts were dried (MgSO4) and the solvent was evaporated to give the crude free amine. Final purification was achieved by high-vacuum bulb-to-bulb distillation.

Following compounds were prepared using this method:
4,10-Dibenzyl-7,16,21-trioxa-1,4,10,13-tetraazabicyclo[11.5.5]tricosane (FIG 1, L11.36)
   Yield: 87 %; 1H-NMR (60 MHz, CDCl3): δ (ppm) = 2.65-2.85. (m, 20 H), 3.59-3.79 (m, 16 H), 7.29-7.49 (m, 10 H); MS (ESI, MeOH): m/z = 511.6 [M+H]+, 533.4 [M+Na]+
4,10-Dipropyl-7,16,21-trioxa-1,4,10,13-tetraazabicyclo[11.5.5]tricosane (FIG 1, L11.58)
   Yield: 92 %; 1H-NMR (60 MHz, CDCl3): δ (ppm) = = 0.63-1.07 (t, 6 H), 1.13-1.62 (m, 4 H), 2.19-3.03 (m, 24 H), 3.27-3.95 (m, 12 H); MS (ESI, MeOH): m/z = 415.5 [M+H]+, 437.3 [M+Na]+

### N-alkylation with oxiranes

Oxa-tetraazabicycloalkane (1 eq, 0.5 mmol), dissolved in anhydrous THF (50 ml), was treated with the solution of hexafluoroisobutene oxide (2 eq. 1 mmol) in 10 ml of anhydrous THF at 0 °C. The reaction mixture was allowed to warm up to RT overnight. The solvent was removed in vacuum, the crude product triturated with hexane, filtered and dried.

Following compounds were prepared using this method:
1,1,1,3,3,3-Hexafluoro-2-({10-[3,3,3-trifluoro-2-hydroxy-2-(trifluoromethyl)propyl]-7,16,21-trioxa-1,4,10,13-tetraazabicyclo[11.5.5]tricos-4-yl}methyl)-2-propanol (FIG 1, L11.57)
Yield:78.6 %; 1H-NMR (60 MHz, CDCl3): δ (ppm) = 2.5-3.2 (m, 24 H), 3.3-3.8 (m, 12 H); MS (ESI): m/z = 691.7 [M+H]+
1,1,1,3,3,3-Hexafluoro-2-({10-[3,3,3-trifluoro-2-hydroxy-2-(trifluoromethyl)propyl]-7,16, 19,24-tetraoxa-1,4, 10, 13-tetraazabicyclo[11.8.5]hexacos-4-yl}methyl)-2-propanol (FIG 1, L12.50)

General procedures for preparation of metal organic compounds:
The Lanthanide source (1 eq) and Ligand (1.05 eq) were dissolved separately in an appropriate solvent (THF or methanol). The solution, containing Eu(II), was added dropwise to the stirred solution of the ligand at RT. If THF only is used as the reaction solvent, the precipitated product is separated by filtration, washed with THF, hexane and dried. In other cases, reaction mixture is evaporated to dryness, residue is triturated with THF or diethyl ether, precipitated product is separated by filtration, washed with THF (diethyl ether), hexane and dried.

| **Name** | **Ligand** | **Lanthanide source** | **Peak of emission (nm)** | **Emission spectrum** |
|---|---|---|---|---|
| E1 | L11.1 | EuI₂ | 489 (MeOH) | FIG. 8, solid line |
| E2 | L11.17 | EuI₂ | 456 (MeOH) | FIG. 8, dashed line |
| E3 | L11.17 | EuBr₂ | 456 (MeOH) | FIG. 8, dotted line |
| E4 | L11.17 | EuCl₂ | 456 (MeOH) | FIG. 8, dash-dotted line |
| E8 | L11.57 | Eu (HMDS)₂ | 529 (MeOH) | FIG. 9, solid line |
| E11 | L12.1 | EuI₂ | 472 (MeOH) | FIG. 12, solid line |
| E12 | L12.17 | EuI₂ | 460 (MeOH) | FIG. 9, dashed line |
| E13 | L12.1 | EuBr₂ | 472 (MeOH) | FIG. 12, dashed line |
| E14 | L12.21 | EuI₂ | 405 (MeOH) | FIG. 9, dotted line |
| E16 | L12.25 | EuI₂ | 442 (MeOH) | FIG. 9, dashed-dotted line |
| E17 | L12.25 | EuBr₂ | 442 (MeOH) | FIG. 10, solid line |
| E18 | L12.25 | EuCl₂ | 442 (MeOH) | FIG. 10, dashed line |
| E19 | L12.49 | Eu (HMDS)₂ | 468 (ACN) | FIG. 10, dotted line |
| E20 | L12.25 | Eu (CB)₂ | green (MeOH) | |
| E22 | L11.1 | CeI₃ | 400 (DCE) | FIG. 10, dashed-dotted line |
| E23 | L11.17 | Eu(BF₄)₂ | 456 (MeOH) | FIG. 11, solid line |
| E24 | L11.17 | Eu(TPB)₂ | Not soluble, green (solid) | |
| E26 | L11.58 | EuI₂ | 456 (MeOH) | FIG. 11, dashed line |
| E27 | L11.36 | EuI₂ | 454 (MeOH) | FIG. 11, dotted line |
| E29 | L11.60 | EuI₂ | 487 (MeOH) | FIG. 12, dotted line |
| E30 | L11.17 | Eu(Prz)2 | 492 (solid) | FIG. 12, dash-dotted line |
| E41 | L11.17 | Eu(BH₄)₂×(THF)ₙ | 456 (MeOH) | FIG. 11, dashed-dotted line |
| E42 | L11.58 | EuBr₂ | 452 (MeOH) | FIG. 13, solid line |
| E43 | L12.17 | Eu(3,5-DiMePrz) | 461 MeOH) | FIG. 13, dashed line |
| E44 | L11.59 | EuI₂ | 456(MeOH) | FIG. 13, dotted line |
| E45 | L11.1 | Eu(Idz)₂ | 490 (MeOH) | FIG. 13, dash-dotted line |
| E46 | L11.17 | Eu(Idz)₂ | 456 (MeOH) | FIG. 14, solid line |
| E47 | L12.9 | Eu(Idz)₂ | 464 (MeOH) | FIG. 14, dashed line |
| E48 | L12.25 | Eu(Idz)₂ | 443 (MeOH) | FIG. 14, dotted line |
| E49 | L12.9 | EuI₂ | 464 (MeOH) | FIG. 14, dash-dotted line |
| E50 | L12.25 | Eu(TFSI)₂ | 443 (MeOH) | FIG. 15, solid line |
| E51 | L11.17 | Eu(TFSI)₂ | 456 (MeOH) | FIG. 15, dashed line |
| E52 | L11.17 | Eu(PFP)₂ | 456 (MeOH) | FIG. 15, dotted line |

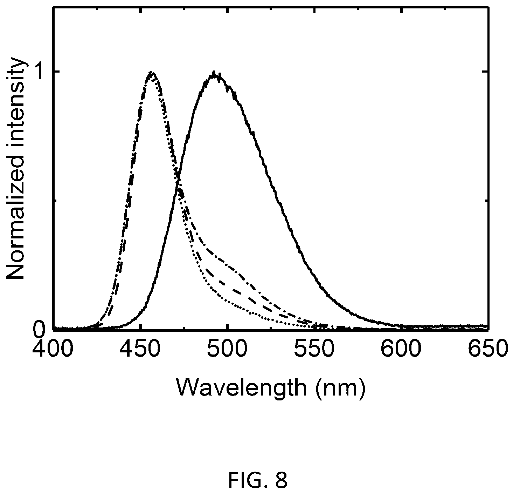

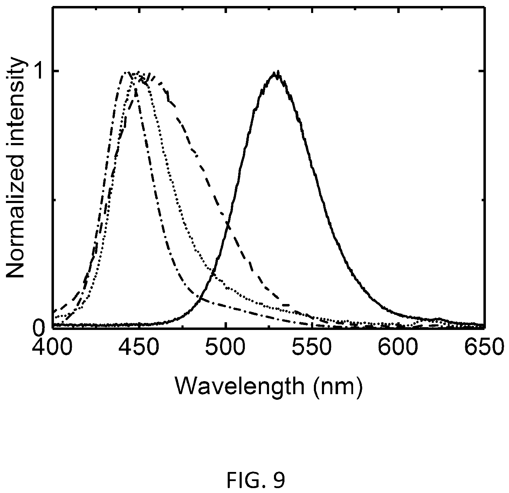

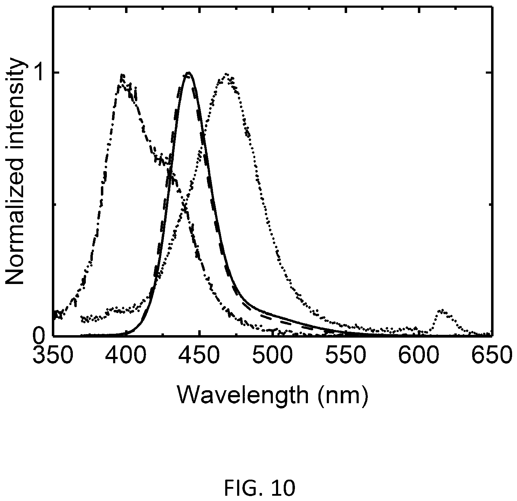

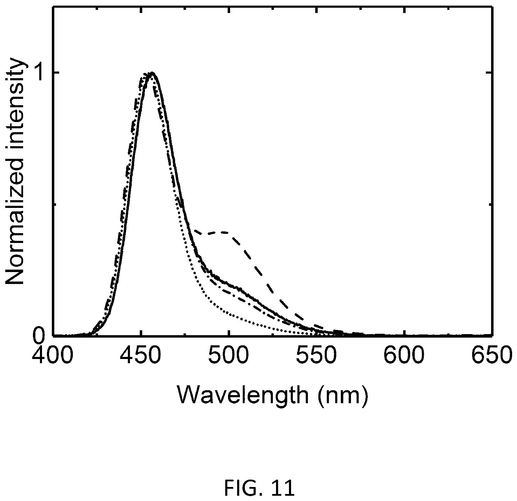

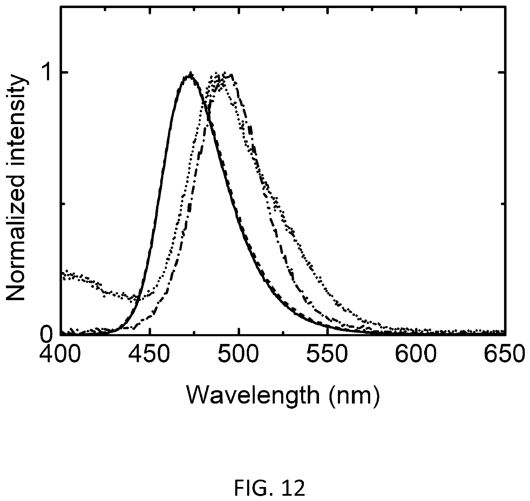

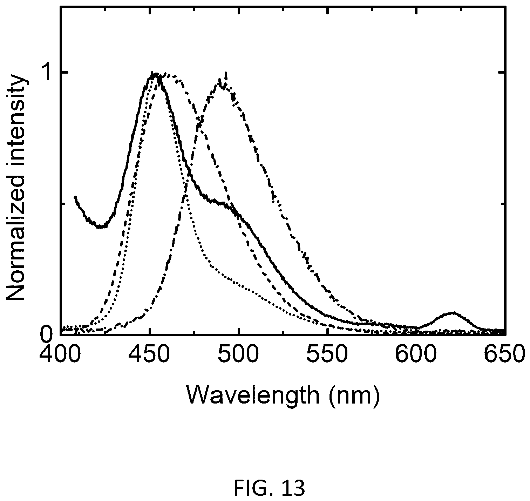

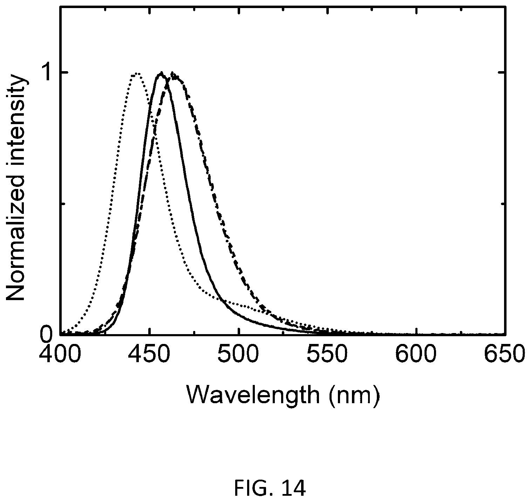

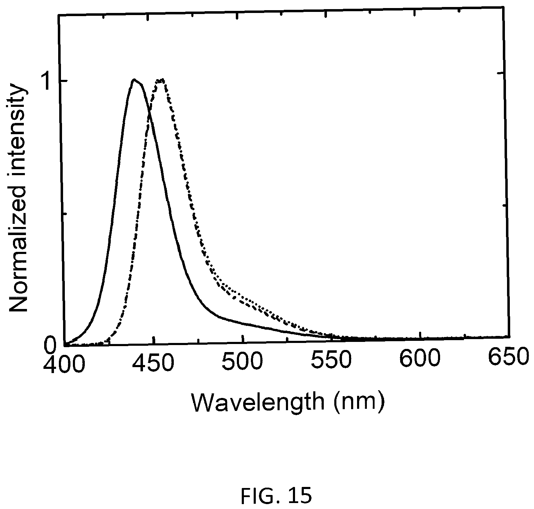

The table above lists synthesized examples according to invention. Please refer to FIG. 5 for the chemical structure of the title compounds (abbreviated with EXX). Accordingly, the ligands are depicted in FIG. 1. Emission spectra of most examples were measured in solution, except for E24, and E30, which were not soluble. The peak emission is given in the table above together with the solvent - most of the examples systems emit in the deep blue spectral region, well suitable for display applications. Finally, the right most column indicates the link to the corresponding emission spectrum, depicted in **FIG 8** to **FIG. 15****.** With reference to those recorded spectra many examples systems show narrow bandwidth emission, which is highly desired for display applications.

### Device examples according to the present invention

A representative embodiment of an organic electronic device according to various embodiments will now be described, including a detailed description of the fabrication process of the organic electronic device. Yet, it will be understood that neither the specific techniques for fabrication of the device, nor the specific device layout, nor the specific compounds are intended to limit the scope of the present invention.

Material definitions:
- ITO:: Indium tin oxide, transparent anode
- MoO3:: Molybdenum trioxide
- NPB:: 4,4'-bis[N-(1-naphthyl)-N-phenylamino]biphenyl
- TAPC:: 4,4'-(1,1-Cyclohexanediyl)bis[N,N-bis(4-methylphenyl)aniline]
- TCTA:: Tris(4-carbazoyl-9-ylphenyl)amine
- mCP:: 1,3-bis(9-carbazolyl)benzene
- mCP-t-Bu:: 1,3-Bis(3,6-di-tert-butyl-9H-carbazol-9-yl)benzene
- TSPO1:: Diphenyl[4-(triphenylsilyl)phenyl]phosphine oxide
- Bphen:: Bathophenanthroline
- Cs:: Cesium
- AI:: Aluminium

NPB, TAPC, TCTA, mCP, mCP-t-Bu, TSPO1, Bphen were purchased from Lumtec and used as-received.

The organic electronic devices were prepared with the following layer sequencees: Device 1

ITO / MoO3 2 nm / NPB 50 nm / TAPC 10 nm / TAPC:E1 15 wt% 20 nm / TSPO1 10 nm / BPhen 30 nm / Cs 2 nm / Al 100 nm.

### Device 2

ITO / MoO3 2 nm / TCTA 30 nm / mCP-t-Bu 10 nm / mCP-t-Bu:eE2 10 wt% 20 nm / TSPO1 10 nm / BPhen 40 nm / Cs 2 nm / Al 100 nm.

### Device 3

ITO / MoO3 2 nm / TCTA 30 nm / mCP 10 nm / mCP-t-Bu:E51 10 wt% 20 nm / TSPO1 10 nm / BPhen 40 nm / Cs 2 nm / Al 100 nm.

The organic electronic devices were each fabricated on a 1"x1" size glass substrate, pre-coated with a transparent ITO anode. The substrates were cleaned with various solvents in an ultrasonic bath and subsequently exposed to oxygen plasma for 10 min. Following the cleaning procedure, the substrates were loaded into an ultra-high vacuum evaporation tool operating at around 10⁻⁷ mbar pressure. Here, the organic layers were deposited onto the substrates in the sequence stated above. In the case of the layer containing an emitter, a co-evaporation process was used in which both materials stated evaporating at the same time at different rates, corresponding to the resulting doping ratio. Each device was finished by evaporating a 100 nm film of cathode Al. The Al deposition was carried out using a structured shadow mask, such that the resulting overlap of pre-structured ITO, organic layers and Al gave an active area of 6.25 mm². Subsequently, the devices were transferred to a nitrogen atmosphere glovebox and encapsulated by glueing an additional encapsulating glass substrate on top to prevent oxygen- and water-induced degradation. The electrical and electroluminescent properties of the devices were measured using a Keithley 2400 source meter, OceanOptics USB4000 spectrometer and a calibrated Si photodiode.

Device 1 showed turn-on at 4.2 V, the current density reached 1 mA/cm² at 7.8 V and 10 mA/cm² at 9.8 V. Device 2 showed turn-on at 3.5 V, the current density reached 1 mA/cm² at 5 V and 10 mA/cm² at 6.1 V. Device 3 showed turn-on at 5.5 V, the current density reached 1 mA/cm² at 7.3 V and 10 mA/cm² at 9.3 V. These values are representative of the typical operating conditions for an organic electronic device used in flat-panel displays.

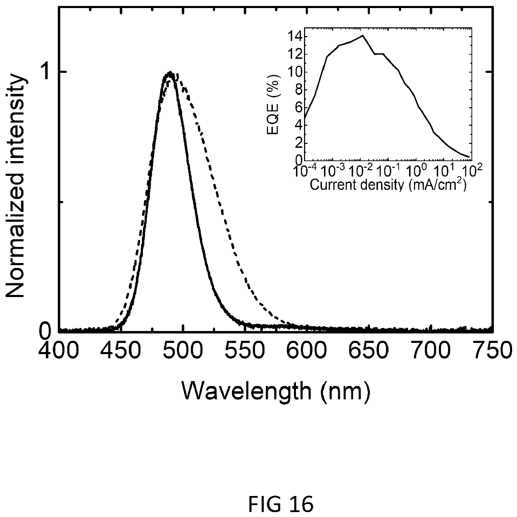

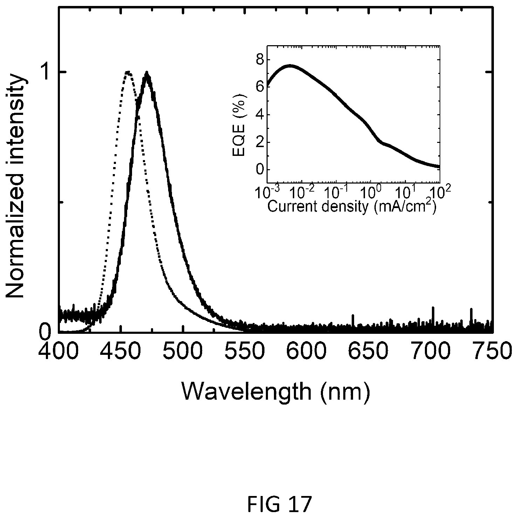

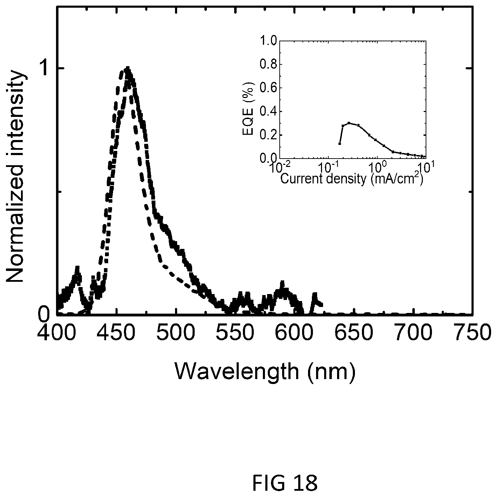

The electroluminescence (dashed line) and photoluminescence measured in MeOH (solid line) spectra of the devices 1-3 are presented in **FIG 16****,** **FIG 17****,** **FIG 18****,** respectively. Evidently, the electroluminescence resembles the photoluminescence very well and all devices emit in the deep blue spectral region, and therefore are suitable for flat panel display applications. The external quantum efficiency curves are plotted in the inset of the three figures for each device.

## Claims

1. A metal-organic coordination compound, wherein the coordination compound comprises one lanthanide ion coordinated by a polycyclic organic ligand having the formula 1-1 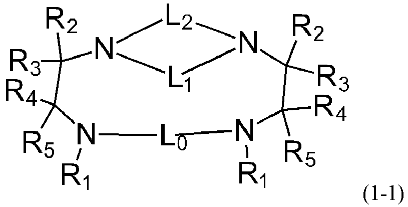 with
• **L₀, L₁**, **L₂** being independently each a divalent organic group formed by removing two hydrogen atoms from a substituted or non-substituted alkane, arylalkane, heteroarylalkane, arene, heteroarene, alkylarene, alkylheteroarene, dialkylarene or dialkylheteroarene, wherein alkane or alkyl can be interrupted by one or more oxygen or boron atoms, and with
• the sum of the number of atoms in the shortest sequences of atoms in **L₁**, and **L₂** between the two linkages of -L₁- and -L₂- and the remainder of the polycyclic ligand being less than 16 atoms, and
• **R₂** to **R₅** independently in each occurrence representing hydrogen, deuterium, halogen, or an organyl group, and
• **R₁** representing hydrogen, deuterium, or an organyl group.

2. The coordination compound according to claim 1, wherein in the ligand **R₂** to **R₅** are hydrogen according to formula 1-2

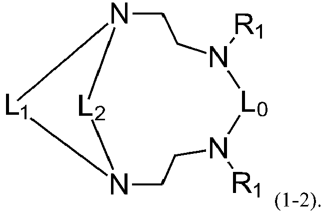

3. The coordination compound according to claim 1 or 2, wherein **L₀** is a substituted or non-substituted 2 to 9 membered linear aliphatic hydrocarbon chain in which one aliphatic chain member can be replaced by a hetero atom selected from N, B, P, O, S, preferably from N and O, more preferably the hetero atom being O.

4. The coordination compound according to any of claims 1 to 3, wherein **L₁,** and **L₂** are comprised of ethylene fragments or ethylene and ethyleneoxy fragments and the ligand has one of formulae 1-3-1 to 1-3-4

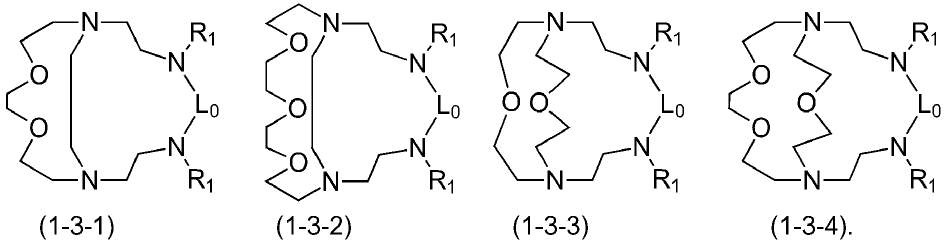

5. The coordination compound according to any of claims 1 to 3, wherein at least two atoms present in the shortest sequence of atoms of either **L₀, L₁,** or **L₂** are part of a benzene, pyridine, pyrrole, pyrazole, furan, carbazole, dibenzofurane or benzofuran ring structure.

6. The coordination compound according to any of claims 1 to 5, wherein the one lanthanide ion is selected from Eu(II), Ce(III), and Yb(II), preferably from Eu(II), and Ce(III), more preferably the lanthanide being Eu(II).

7. The coordination compound according to any of claims 1 to 6, wherein the coordination compound comprises at least one negatively charged anion that is not covalently linked to the polycyclic organic ligand.

8. The coordination compound according to claim 7, wherein the at least one negatively charged anion comprises of more than 2 atoms or has a molecular weight of more than 128g/mole.

9. The coordination compound according to any of claims 1 to 8, wherein **R₁** is H, deuterium, substituted or unsubstituted C₁₋₁₂-alkyl, aryl, aralkyl, heteroaralkyl, or alkaryl, and wherein the two **R₁** can be covalently linked with each other.

10. The coordination compound according to any of claims 1 to 9 having a structure according to formula (1-4) 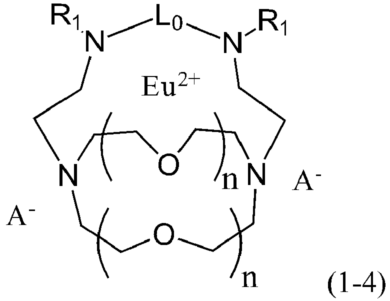 wherein
• **n** independently in each occurrence is 0, 1, 2, or 3, preferably 1 or 2, whilst the sum of both **n** is either 2 or 3 and
• **R₁** is H, deuterium, substituted or unsubstituted C₁₋₁₂-alkyl, aryl, aralkyl, heteroaralkyl, or alkaryl, and wherein the two **R₁** can be covalently linked with each other to form a cyclic group, and
• **L₀** is a substituted or non-substituted 2 to 9 membered linear aliphatic hydrocarbon chain in which one aliphatic chain member can be replaced by a hetero atom selected from N, B, P, O, S, preferably from N and O, more preferably the hetero atom being O, and
• **A-** is a singly negatively charged anion.

11. The coordination compound according to any of claims 1 to 9, wherein the polycyclic organic ligand is at least singly negatively charged.

12. The coordination compound according to claim 11, wherein R₁ is selected from: 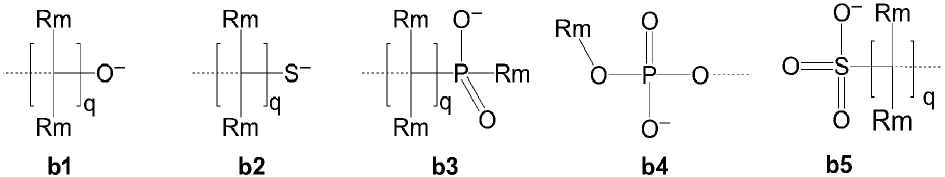 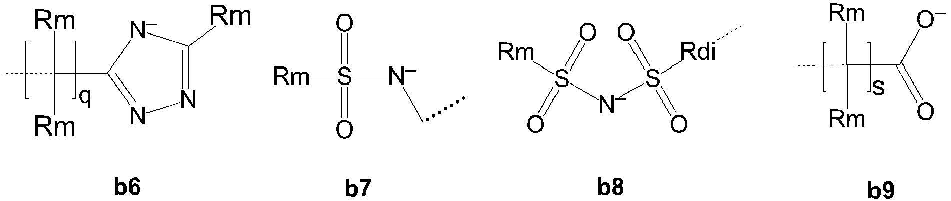 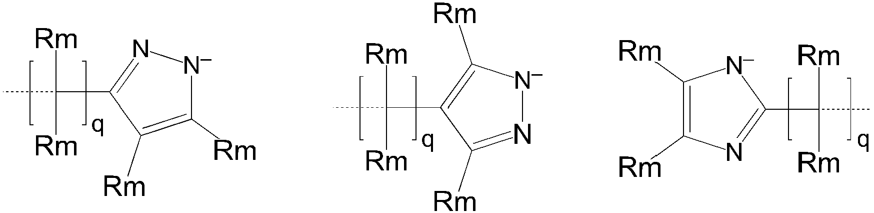 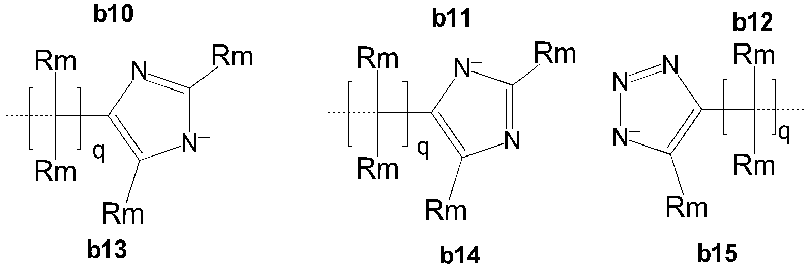 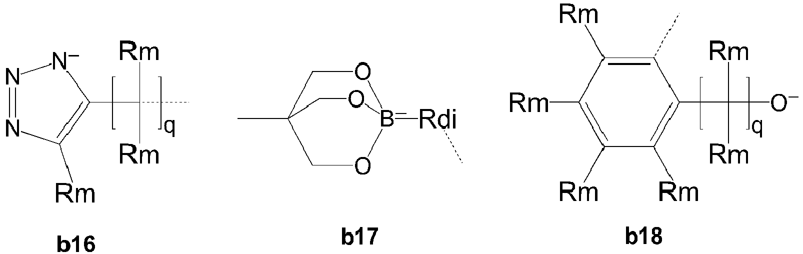 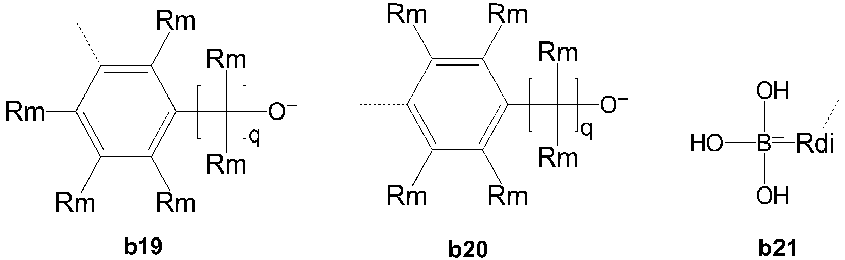 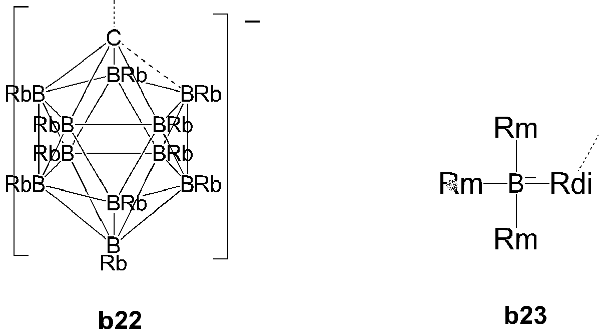 wherein
• the dashed lines indicate the linkage to the nitrogen atoms in N-R₁, and
• **R_{b}** independently in each occurrence represents either hydrogen, halogene, or methyl
• **Rₘ** independently in each occurrence represents a monovalent group formed by removing a hydrogen atom from a substituted or non-substituted alkane, arene, or heteroarene, and
• **R_{di}** represents a divalent group formed by removing two hydrogen atoms from a substituted or non-substituted alkane, arene, or heteroarene, and
• **q** independently in each occurrence is 0, 1, 2, or 3 and
• **s** independently in each occurrence is 1, 2, or 3.

13. A mixture comprising
a second electrically neutral organic compound, and
the coordination compound according to any one of claims 1 to 12, wherein the coordination compound is imbedded into the at least one second electrically neutral organic compound,
wherein the second organic compound has a triplet energy higher than 2.5 eV, preferably higher than 2.6 eV and even more preferably higher than 2.7 eV and/or wherein the coordination compound has a higher hole affinity compared to the second organic compound.

14. A compound comprising
the coordination compound according to any one of claims 1 to 12, and a polymer with a molecular weight Mn above 1000 g/mol, wherein the coordination compound is covalently attached to the polymer backbone.

15. An organic electronic device, comprising:
a first electrode;
a second electrode; and
an organic layer arranged such that it is electrically interposed between the first and second electrodes, wherein the organic layer comprises the coordination compound according to any one of claims 1 to 12, the mixture of claim 13 or the compound of claim 14, wherein preferably the organic electronic device is an organic light emitting diode.

16. A method of forming an organic device, the method comprising:
forming a layer of the coordination compound according to any one of claims 1 to 12, of the mixture of claim 13 or the compound of claim 14,
wherein the layer is deposited from a gas phase, in particular using an evaporation and/or sublimation and/or carrier gas process, wherein preferably the coordination compound according to any one of claims 1 to 12 is transferred into gas phase from a blend of the coordination compound with at least one second material, with the second material is present in the blend in an amount of between 1 and 99 vol%.

17. The use of a metal-organic coordination compound according to one of claims 1 to 12 in an organic electronic device, as a dye or as a contrast enhancement medium for magnetic resonance tomography, or of a polycyclic organic ligand as defined in one of claims 1 to 12 for extracting three-valent actinides from radioactive waste water.

## Amended claims

### Amended claims in accordance with Rule 137(2) EPC.

1. A metal-organic coordination compound, wherein the coordination compound comprises one lanthanide ion coordinated by a polycyclic organic ligand having the formula 1-1 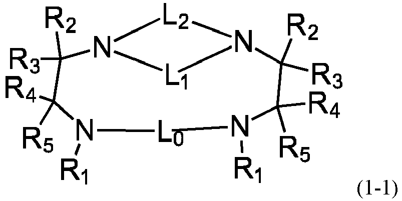 with
• **L₀, L₁, L₂** being independently each a divalent organic group formed by removing two hydrogen atoms from a substituted or non-substituted alkane, arylalkane, heteroarylalkane, arene, heteroarene, alkylarene, alkylheteroarene, dialkylarene or dialkylheteroarene, wherein alkane or alkyl can be interrupted by one or more oxygen or boron atoms, and wherein the substituents are selected from the group consisting of deuterium, halogen, alkyl, cycloalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and preferably are selected from the group consisting of deuterium, alkyl, cycloalkyl, aryl, heteroaryl and with
• the sum of the number of atoms in the shortest sequences of atoms in **L_{1,}** and **L₂** between the two linkages of -L₁- and -L₂- and the remainder of the polycyclic ligand being less than 16 atoms, and
• **R₂** to **R₅** independently in each occurrence representing hydrogen, deuterium, halogen, or an organyl group, and
• **R₁** representing hydrogen, deuterium, or an organyl group, which is any chemically stable organic arrangement of atoms where one hydrogen atom has been removed such as to use this free valance to covalently link the organic group.

2. The coordination compound according to claim 1, wherein in the ligand **R₂** to **R₅** are hydrogen according to formula 1-2

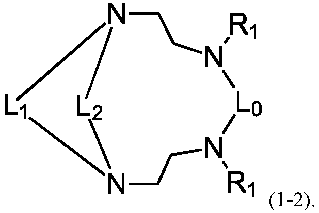

3. The coordination compound according to claim 1 or 2, wherein **L₀** is a substituted or non-substituted 2 to 9 membered linear aliphatic hydrocarbon chain in which one aliphatic chain member can be replaced by a hetero atom selected from N, B, P, O, S, preferably from N and O, more preferably the hetero atom being O.

4. The coordination compound according to any of claims 1 to 3, wherein **L_{1,}** and **L₂** are comprised of ethylene fragments or ethylene and ethyleneoxy fragments and the ligand has one of formulae 1-3-1 to 1-3-4

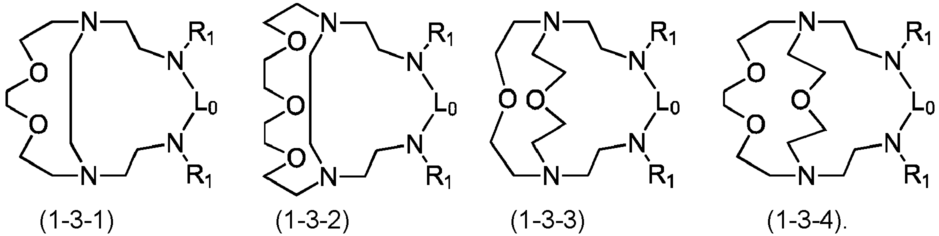

5. The coordination compound according to any of claims 1 to 3, wherein at least two atoms present in the shortest sequence of atoms of either **L₀**, **L₁**, or **L₂** are part of a benzene, pyridine, pyrrole, pyrazole, furan, carbazole, dibenzofurane or benzofuran ring structure.

6. The coordination compound according to any of claims 1 to 5, wherein the one lanthanide ion is selected from Eu(II), Ce(III), and Yb(II), preferably from Eu(II), and Ce(III), more preferably the lanthanide being Eu(II).

7. The coordination compound according to any of claims 1 to 6, wherein the coordination compound comprises at least one negatively charged anion that is not covalently linked to the polycyclic organic ligand.

8. The coordination compound according to claim 7, wherein the at least one negatively charged anion comprises of more than 2 atoms or has a molecular weight of more than 128g/mole.

9. The coordination compound according to any of claims 1 to 8, wherein **R₁** is H, deuterium, substituted or unsubstituted C₁₋₁₂-alkyl, aryl, aralkyl, heteroaralkyl, or alkaryl, and wherein the two **R₁** can be covalently linked with each other, , and wherein the substituents are selected from the group consisting of deuterium, halogen, alkyl, cycloalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and preferably are selected from the group consisting of deuterium, alkyl, cycloalkyl, aryl, heteroaryl.

10. The coordination compound according to any of claims 1 to 9 having a structure according to formula (1-4) 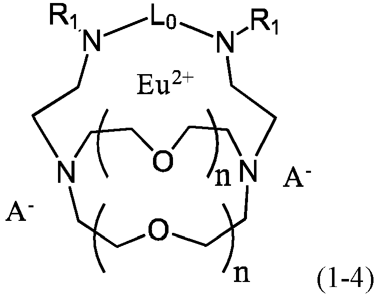 wherein
• **n** independently in each occurrence is 0, 1, 2, or 3, preferably 1 or 2, whilst the sum of both **n** is either 2 or 3 and
• **R₁** is H, deuterium, substituted or unsubstituted C₁₋₁₂-alkyl, aryl, aralkyl, heteroaralkyl, or alkaryl, and wherein the two **R₁** can be covalently linked with each other to form a cyclic group, and wherein the substituents are selected from the group consisting of deuterium, halogen, alkyl, cycloalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and preferably are selected from the group consisting of deuterium, alkyl, cycloalkyl, aryl, heteroaryl, and
• **L₀** is a substituted or non-substituted 2 to 9 membered linear aliphatic hydrocarbon chain in which one aliphatic chain member can be replaced by a hetero atom selected from N, B, P, O, S, preferably from N and O, more preferably the hetero atom being O, and
• **A-** is a singly negatively charged anion.

11. The coordination compound according to any of claims 1 to 9, wherein the polycyclic organic ligand is at least singly negatively charged.

12. The coordination compound according to claim 11, wherein R₁ is selected from: 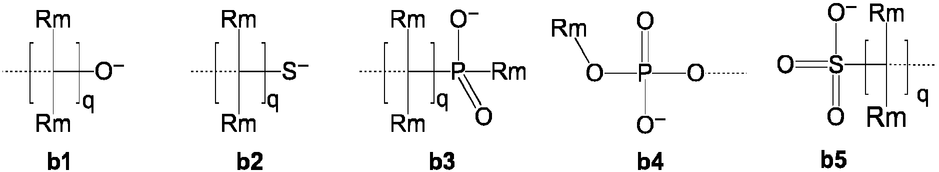 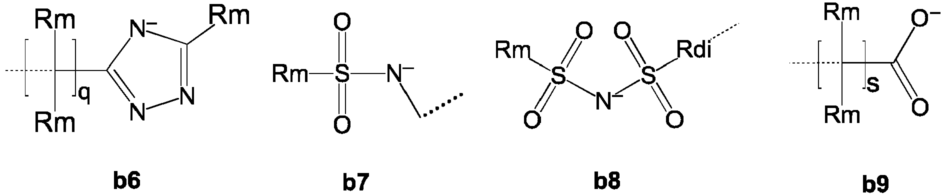 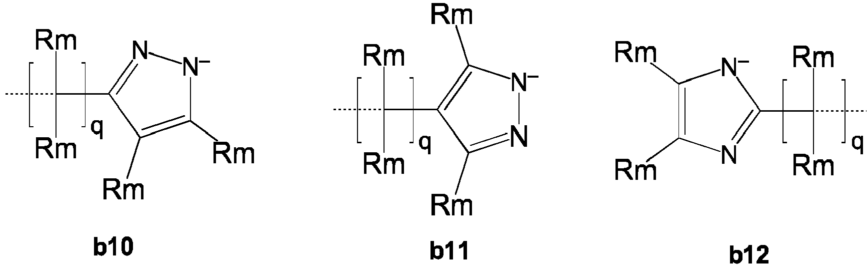 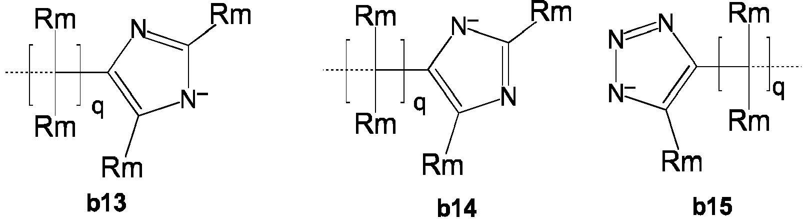 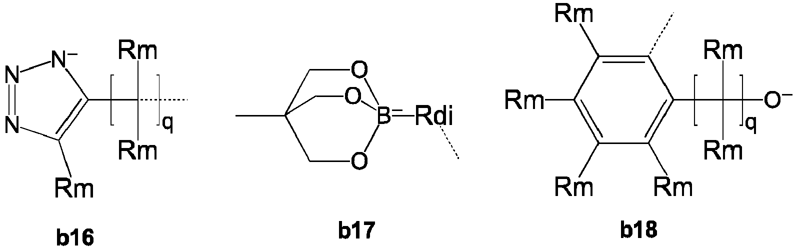 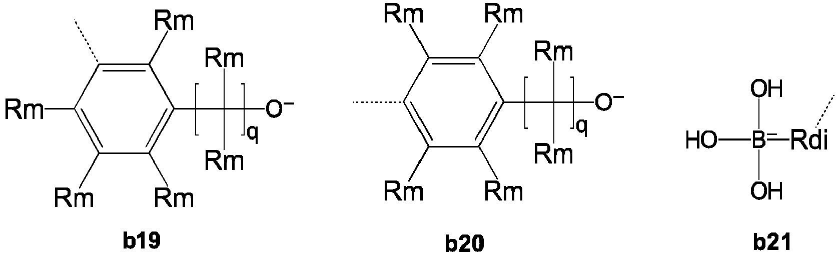 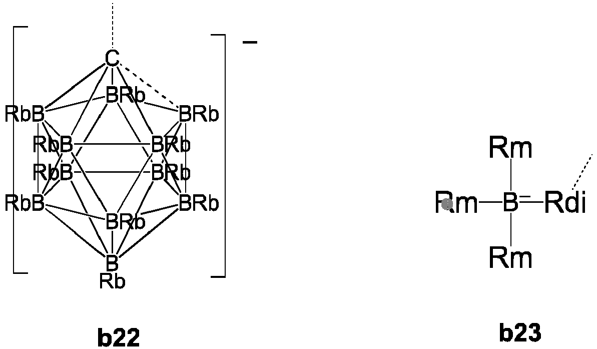 wherein
• the dashed lines indicate the linkage to the nitrogen atoms in N-R₁, and
• **R_{b}** independently in each occurrence represents either hydrogen, halogene, or methyl
• **Rₘ** independently in each occurrence represents a monovalent group formed by removing a hydrogen atom from a substituted or non-substituted alkane, arene, or heteroarene, and
• **R_{di}** represents a divalent group formed by removing two hydrogen atoms from a substituted or non-substituted alkane, arene, or heteroarene, and
• **q** independently in each occurrence is 0, 1, 2, or 3 and
• **s** independently in each occurrence is 1, 2, or 3, and wherein the substituents are selected from the group consisting of deuterium, halogen, alkyl, cycloalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and preferably are selected from the group consisting of deuterium, alkyl, cycloalkyl, aryl, heteroaryl.

13. A mixture comprising
a second electrically neutral organic compound, and
the coordination compound according to any one of claims 1 to 12,
wherein the coordination compound is imbedded into the at least one second electrically neutral organic compound,
wherein the second organic compound has a triplet energy higher than 2.5 eV, preferably higher than 2.6 eV and even more preferably higher than 2.7 eV and/or wherein the coordination compound has a higher hole affinity compared to the second organic compound.

14. A compound comprising
the coordination compound according to any one of claims 1 to 12, and
a polymer with a molecular weight Mn above 1000 g/mol, wherein the coordination compound is covalently attached to the polymer backbone.

15. An organic electronic device, comprising:
a first electrode;
a second electrode; and
an organic layer arranged such that it is electrically interposed between the first and second electrodes, wherein the organic layer comprises the coordination compound according to any one of claims 1 to 12, the mixture of claim 13 or the compound of claim 14, wherein preferably the organic electronic device is an organic light emitting diode.

16. A method of forming an organic device, the method comprising:
forming a layer of the coordination compound according to any one of claims 1 to 12, of the mixture of claim 13 or the compound of claim 14,
wherein the layer is deposited from a gas phase, in particular using an evaporation and/or sublimation and/or carrier gas process, wherein preferably the coordination compound according to any one of claims 1 to 12 is transferred into gas phase from a blend of the coordination compound with at least one second material, with the second material is present in the blend in an amount of between 1 and 99 vol%.

17. The use of a metal-organic coordination compound according to one of claims 1 to 12 in an organic electronic device, as a dye or as a contrast enhancement medium for magnetic resonance tomography, or of a polycyclic organic ligand as defined in one of claims 1 to 12 for extracting three-valent actinides from radioactive waste water.
